(19) **Europäisches Patentamt**
**European Patent Office**
**Office européen des brevets**

(11) **EP 3 147 822 B1**

(12) **FASCICULE DE BREVET EUROPEEN**

(45) Date de publication et mention de la délivrance du brevet:
**03.03.2021 Bulletin 2021/09**

(51) Int Cl.:
*G06K 9/00* (2006.01)     *A61B 5/1172* (2016.01)
*G01B 9/02* (2006.01)

(21) Numéro de dépôt: **16189504.0**

(22) Date de dépôt: **19.09.2016**

(54) **PROCEDE D'EXTRACTION DE CARACTERISTIQUES MORPHOLOGIQUES D'UNE EMPREINTE DIGITALE**

VERFAHREN ZUR EXTRAKTION MORPHOLOGISCHER MERKMALE EINES FINGERABDRUCKS

METHOD FOR EXTRACTING MORPHOLOGICAL CHARACTERISTICS OF A FINGERPRINT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.09.2015 FR 1558929**

(43) Date de publication de la demande:
**29.03.2017 Bulletin 2017/13**

(73) Titulaire: **Safran Identity & Security**
**92130 Issy-les-Moulineaux (FR)**

(72) Inventeurs:
• **LAMARE, François**
  **91140 VILLEBON-SUR-YVETTE (FR)**
• **GOTTESMAN, Yaneck**
  **92370 CHAVILLE (FR)**
• **DORIZZI, Bernadette**
  **75015 PARIS (FR)**

(74) Mandataire: **Nony**
**11 rue Saint-Georges**
**75009 Paris (FR)**

(56) Documents cités:
**US-A1- 2014 241 596     US-A1- 2015 168 127**

• **KHUTLANG RETHABILE ET AL: "Novelty Detection-Based Internal Fingerprint Segmentation in Optical Coherence Tomography Images", 2014 SECOND INTERNATIONAL SYMPOSIUM ON COMPUTING AND NETWORKING, IEEE, 10 décembre 2014 (2014-12-10), pages 556-559, XP032741753, DOI: 10.1109/CANDAR.2014.73**

• **LUKE NICHOLAS DARLOW ET AL: "Internal fingerprint zone detection in optical coherence tomography fingertip scans", JOURNAL OF ELECTRONIC IMAGING., vol. 24, no. 2, 9 avril 2015 (2015-04-09), page 023027, XP055273786, 1000 20th St. Bellingham WA 98225-6705 USA ISSN: 1017-9909, DOI: 10.1117/1.JEI.24.2.023027**

• **MENGYANG LIU ET AL: "Biometric Mapping of Fingertip Eccrine Glands With Optical Coherence Tomography", IEEE PHOTONICS TECHNOLOGY LETTERS, IEEE SERVICE CENTER, PISCATAWAY, NJ, US, vol. 22, no. 22, 15 novembre 2010 (2010-11-15), pages 1677-1679, XP011319083, ISSN: 1041-1135**

• **BOSSEN A ET AL: "Internal Fingerprint Identification With Optical Coherence Tomography", IEEE PHOTONICS TECHNOLOGY LETTERS, IEEE SERVICE CENTER, PISCATAWAY, NJ, US, vol. 22, no. 7, 1 avril 2010 (2010-04-01), pages 507-509, XP011301136, ISSN: 1041-1135**

• **OTHMAN NADIA ET AL: "Impact of Quality-Based Fusion Techniques for Video-Based Iris Recognition at a Distance", IEEE TRANSACTIONS ON INFORMATION FORENSICS AND SECURITY, IEEE, PISCATAWAY, NJ, US, vol. 10, no. 8, 1 août 2015 (2015-08-01), pages 1590-1602, XP011584579, ISSN: 1556-6013, DOI: 10.1109/TIFS.2015.2421314 [extrait le 2015-06-17]**

**Description**

La présente invention concerne un procédé d'extraction de caractéristiques morphologiques de matériels biologiques, en particulier des empreintes digitales, notamment internes ou externes, utilisant des signaux délivrés par des dispositifs d'acquisition par tomographie optique cohérente, notamment pour la biométrie.

La technique d'imagerie tomographique optique cohérente (OCT) est une technique d'imagerie optique sans contact puissante qui est actuellement couramment utilisée dans le secteur médical. Elle commence à être utilisée pour des applications grand public, notamment pour des applications biométriques. Les problématiques propres à ce type d'applications s'avèrent actuellement différentes et étroitement liées à l'étude de propriétés de surfaces délimitées à partir d'informations natives tridimensionnelles.

Par construction, un dispositif d'imagerie SW-OCT est un dispositif interférentiel réalisé à partir d'un interféromètre (de type Michelson ou Mach-Zender) et une source accordable. Chaque mesure consiste en l'enregistrement du signal interférométrique en fonction de la fréquence de la source. Les signaux complexes (intensité et phase) sont donc nativement enregistrés dans le domaine spectral.

Ces signaux sont usuellement représentés dans le domaine spatial (ou temporel puisque la position peut être ramenée à un temps de vol de la lumière) après transformée de Fourier du signal enregistré. Le signal complexe ainsi obtenu est appelé A-scan.

Pour un objet statique (i.e. non soumis à une déformation temporelle et par ailleurs immobile), au voisinage de chaque centre de diffusion considéré comme achromatique (ou de réflexion), la phase spatiale évolue linéairement avec distance selon l'axe z.

Lorsque l'objet possède des propriétés dynamiques (déformation et/ou déplacement), toute variation de la phase spatiale en des instants de mesures différents est associée à un effet Doppler.

La phase spectrale relative à un centre diffusant évolue linéairement avec la fréquence $\upsilon$ de la source. A un coefficient de proportionnalité près, la pente de la phase dans le domaine spectral, définie par $d\phi_m/d\upsilon$, permet de connaître la position spatiale du centre diffusant (ou le temps de vol mis par la lumière pour atteindre le centre diffusant).

Etat de la technique

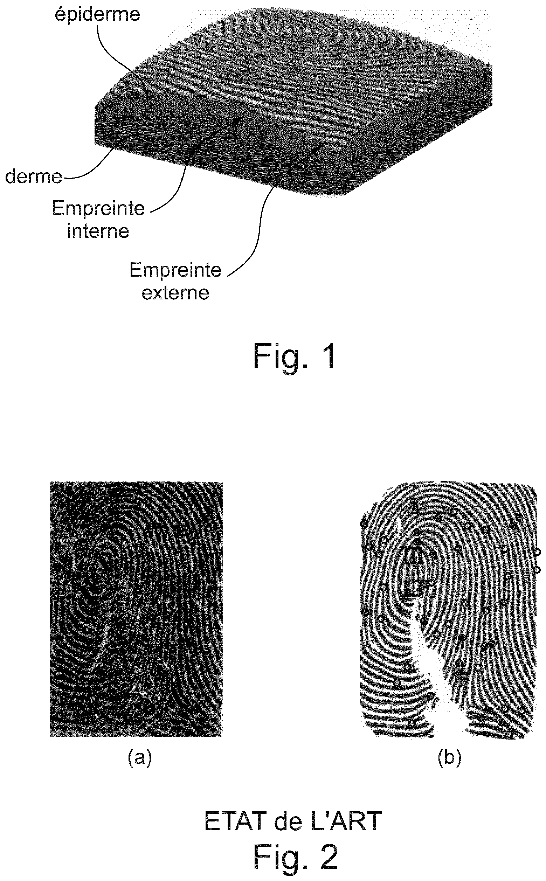

Dans l'étude de la morphologie des différentes couches de matériels biologiques situés sous la peau, comme représenté à la figure 1, les méthodes connues exploitent seulement les informations d'intensité, notamment pour la segmentation d'images en intensité afin de délimiter des surfaces séparant deux matériels biologiques distincts. Cette segmentation est délicate, l'intensité du signal délivré par le capteur OCT étant étroitement dépendante des tissus situés au-dessus du tissu d'intérêt. Cela crée une variabilité dans la segmentation des images utilisées qui nuit à l'extraction de la surface recherchée. Dans le domaine de la biométrie, les images de l'empreinte digitale interne située sous la peau, à l'interface « épiderme / derme » obtenue après segmentation présentent des zones non exploitables avec ce type de capture et ne permettent donc pas toujours une identification des personnes aisée et fiable. Pourtant, les empreintes internes sont mieux préservées que les empreintes externes, ne subissant pas les mêmes altérations de leur propriétés de surface que ces dernières, comme des cicatrices, des tâches, par exemple de l'encre ou de la saleté, ou encore la variation de l'humidité surfacique du doigt, notamment due à la transpiration ou aux conditions d'humidité ambiante. L'empreinte digitale interne est donc une information biométrique très pertinente car plus stable au cours du temps et moins tributaire des variations environnementales. Elle peut par ailleurs permettre l'authentification d'une personne dont l'empreinte digitale externe est abîmée.

L'empreinte digitale interne peut également permettre de détecter une tentative de fraude d'identité. En effet, une méthode de fraude connue, difficile à détecter par les capteurs biométriques connus, consiste à déposer sur le doigt de l'usurpateur une surcouche où est inscrite en relief une empreinte digitale d'une autre personne. Cette surcouche est difficile à détecter, notamment car sous celle-ci se trouve un doigt réel avec du sang oxygéné, et la température de la surcouche est proche de celle de la surface d'un doigt réel.

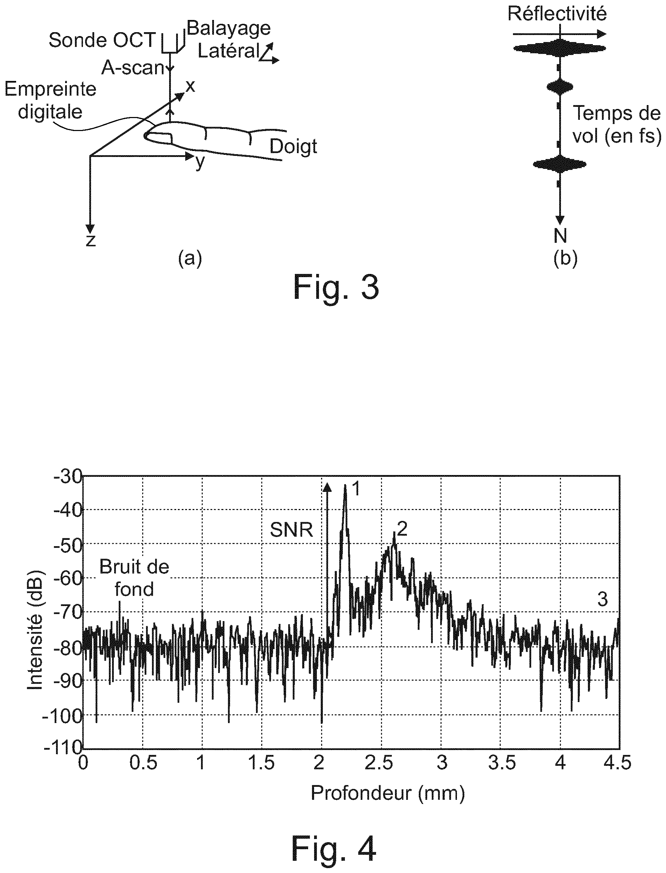

La figure 1 représente une image typique obtenue d'un doigt par tomographie optique cohérente. Sur cette figure, on a représenté le niveau du signal rétrodiffusé en fonction de la position spatiale. Comme représenté à la figure 3(a). la sonde du capteur OCT est déplacée à l'aide de deux miroirs galvanométriques suivant les axes X et Y Pour chaque position de la sonde, une mesure obtenue par interférométrie est enregistrée, comme décrit dans l'article de A. F. Fercher et al. « Optical Coherence Tomography - Principles and Applications », publié dans « Reports on progress in physics », 2003, n°66, pages 239-303. Cela consiste en une mesure de l'intensité rétrodiffusée en fonction du temps de vol, c'est-à-dire le temps que met la lumière à traverser les différentes couches de l'échantillon examiné. La distance de propagation à partir de la sonde peut être trouvée en multipliant le temps de vol par la vitesse de la lumière. Un profil de réflectivité de la lumière en profondeur est alors obtenu, appelé « A-scan » et représenté à la figure 3(b).

Un exemple de profil « A-scan » en intensité d'un doigt est représenté en figure 4. Le signal d'intérêt correspondant au signal provenant de la partie superficielle externe du doigt est compris entre le premier pic numéroté 1 et le troisième pic numéroté 3. Avant le premier pic numéroté 1, seul le bruit de fond est visible. Le pic numéroté 1 correspond à l'interface air/peau, c'est-à-dire à l'empreinte digitale externe. Il s'agit de l'interface où le saut d'indice optique est le plus grand, dû à l'inhomogénéité des deux milieux, induisant l'amplitude plus élevée du pic. En cas de tentative de fraude, le pic numéroté 1 correspond à l'interface air/surcouche. Après ce pic, l'intensité du signal décroit globalement. Cette décroissance est due aux phénomènes d'absorption et de diffusion au fur et à mesure de la pénétration de la lumière dans le tissu ou la surcouche, et donc au cours de sa pénétration dans les différentes couches de la peau ou la surcouche.

La détection de la position du pic d'intensité maximale permet de localiser l'interface air/peau ou air/surcouche. En récupérant la position du maximum de chaque profil « *A-scan* » du volume tomographique, correspondant au temps de vol de la lumière entre la sonde et la surface externe du doigt, on peut construire une surface en trois dimensions, dite surface 3D, associée à l'empreinte digitale externe.

Pour former sélectivement l'image de l'empreinte interne et l'isoler du reste du volume, les méthodes connues reposent sur un filtrage spatial suivant l'axe Z. Ce filtrage permet d'obtenir un niveau moyen de l'intensité rétrodiffusée autour de la profondeur à laquelle se situe l'empreinte interne. La zone de filtrage correspond alors au voisinage spatial du deuxième pic principal de chaque profil « *A-scan* », directement lié à la surface 3D de l'empreinte interne.. L'image ainsi obtenue , dite image en intensité, est présentée à la figure 2(a). Ces méthodes sont décrites notamment dans l'article de A. Bossen et al. « Internai Fingerprint identification with optical coherence tomography », publié dans « IEEE Photonics technology letters », Vol. 22, No. 7, 2010 et l'article de M. Liu et al. « Biometric mapping of fingertip eccrine glands with optical coherence tomography » publié dans « IEEE photonics technology letters », Vol. 22, No. 22, 2010. La figure 2(b) représente l'image traitée par un système logiciel, appelé « *matcher* » en anglais, chargé de mettre l'image sous forme de données binaires et de repérer les minuties de l'empreinte, les points caractéristiques de l'empreinte servant à l'identification. Certaines zones où le contraste de l'empreinte est faible apparaissent en blanc. Pour un même doigt, la position de ces zones peut varier en fonction des conditions expérimentales, ou du positionnement du doigt par rapport au capteur. Les performances obtenues à partir de l'image en intensité peuvent présenter des zones difficiles à exploiter en raison de la qualité des images, comme visible à la figure 2(b).

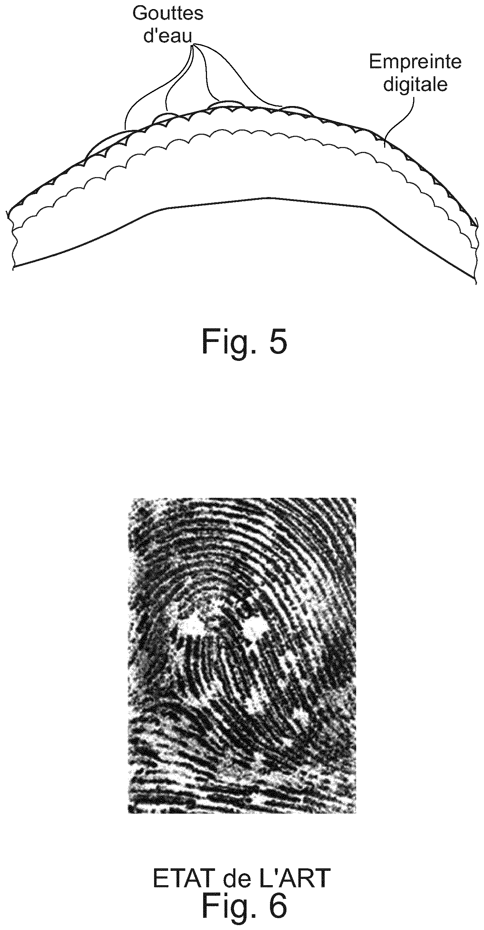

Dans le cas de l'empreinte externe, cette information de contraste d'intensité est en outre très variable en fonction de l'état de la surface du doigt, s'il est tâché, par de l'encre par exemple, ou humide. Les performances en vérification d'empreintes sont notamment très dégradées dans le cas de doigts humides dans des acquisitions effectuées par des capteurs connus, tels que par exemple les capteurs capacitifs ou optiques avec contact, donnant des images en deux dimensions, ou les capteurs optiques sans contact dits « *2 dimensions ½* », comme mentionné dans les articles de R. Cappelli et al. "Performances evaluation of fingerprint vérification systems ", IEEE transactions on pattern analysis and maching intelligence, vol. 28, No. 1, 2006, et de L.C. Jain et al. "Intelligent biometric techniques in fingerprint and face recognition", chapitre 2, Vol. 10, CRC press, 1999. Cette dégradation des performances survient également pour les capteurs biométriques sans contact tel que l'OCT. La figure 5 présente un exemple expérimental d'image OCT en coupe transversale extraite de façon traditionnelle, représentative d'un bout de doigt avec présence de microgouttelettes en sa surface, simulant le comportement d'un doigt en conditions de forte humidité ou de transpiration. L'image en intensité de cet exemple de doigt humide, présentée à la figure 6, contient des taches blanches, dues entre autre à la présence des gouttelettes qui modifient localement les propriétés optiques du doigt, créant un effet de lentille parasite par exemple. Les lecteurs d'empreintes digitales connus visant à authentifier des personnes s'avèrent ainsi non opérationnels dans des conditions d'humidité élevées, aucune image fiable de l'empreinte d'un doigt mouillé ne pouvant être fournie. Dans l'approche présentée dans l'article susmentionné de A. Bossen et al., l'acquisition est réalisée par contact du doigt sur une plaque de verre, conduisant à un aplatissement du doigt.

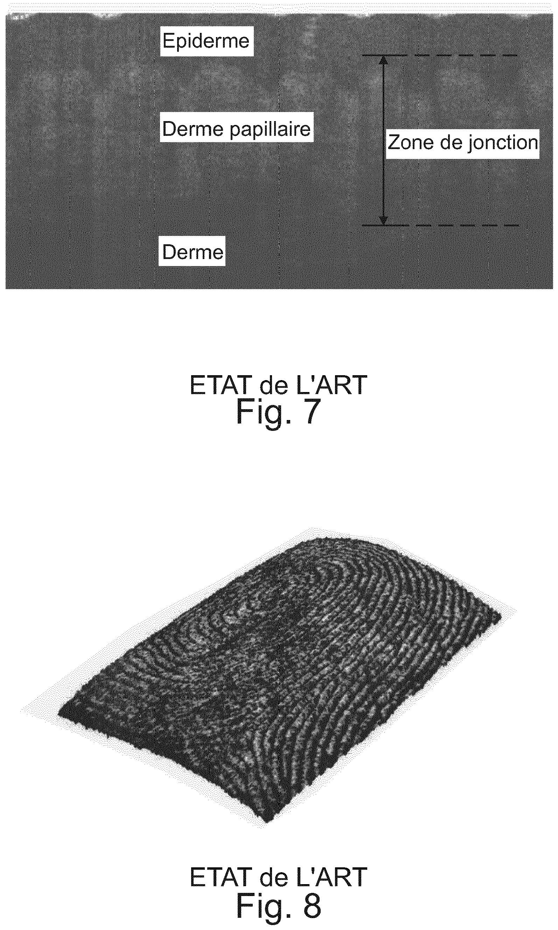

La figure 7 représente une coupe transversale où le derme papillaire, c'est-à-dire l'empreinte interne, est bien visible. Sur cette même figure est représentée la zone de jonction, ou « *junction zone* » en anglais, située entre le derme et l'épiderme. La largeur et la position de cette zone a été fixée empiriquement entre 0.1mm et 0.9mm à l'intérieur du doigt, de telle manière à contenir l'interface de l'empreinte interne. La zone de jonction tridimensionnelle complète peut être obtenue à partir des signaux des différents A-scans $A_{xy}(z)$, pour une position de sonde positionnée en (x,y). Elle contiendra alors toute l'empreinte interne en 3D.

L'image *d'intensité* I(x,y) de l'empreinte interne est alors obtenue en moyennant suivant la direction z le signal $A_{xy}(z)$ sur la zone de jonction :

$$I(x,y) = \frac{1}{\Delta_z} \sum_{z \in J} A_{xy}(z)$$

où x et y correspondent à la position de la sonde, J est la zone de jonction, et $\Delta_z$ la largeur de la zone de jonction. Cette méthode permet d'obtenir une image de textures 2D dite *d'intensité,* notée I(x, y), liée à l'information d'intensité moyenne, x et y étant les coordonnées des pixels de l'image. Des méthodes de traitement d'images ont été appliquées à l'image I(x, y) afin d'améliorer son contraste.

Dans le cas où le doigt n'est pas aplati lors de l'acquisition, l'image I(x, y) peut être projetée sur la surface 3D de l'empreinte interne, obtenue grâce aux mesures de phase. L'empreinte *3D* interne obtenue avec cette méthode est visible à la figure 8. Les performances de cette approche dans le contexte de la biométrie ne sont pas non plus suffisantes en raison de la qualité dégradée des images obtenues. US 2014/0241596 et US 2015/016127 divulguent des procédés visant à représenter les écoulements sanguins dans les réseaux vasculaires du doigt, afin de vérifier que c'est bien l'image d'un doigt vivant qui est acquise. L'information pertinente ici est une information de type Doppler, et il est proposé d'effectuer des différences de phase entre des A-scans (profils de réflectivité en profondeur) successifs. Ce traitement implique nécessairement que la phase en question soit une phase dans le domaine « spatial ». Il n'y a aucune mesure effectuée sur la phase dans le domaine spectral.

L'article Novelty Detection-Based Internai Fingerprint Segmentation in Optical Coherence Tomography Images 2014 Second International Symposium on Computing and Networking Rethabile Khutlang et al divulgue un procédé de segmentation de l'empreinte interne. Les traitements sont effectués B-scan par B-scan (coupes transversales) et non pas A-scan par A-scan (profil de l'intensité rétrodiffusée suivant la profondeur, ou de manière équivalente suivant le temps de vol). L'empreinte n'est ainsi pas segmentée à partir de mesures de distances sur des A-scans (traitement du signal), mais grâce à des méthodes de clustering (GMMs et k-means) appliquées aux B-scans (traitement d'images). Il n'y a aucune mention de mesures de distances via la phase dans le domaine spectral.

L'article Internai fingerprint zone détection in optical coherence tomography fingertip scans Journal of electronic Imaging 24(2) Mar/apr 2015 a également pour finalité une segmentation de l'empreinte interne. Premièrement, l'interface de l'empreinte interne est grossièrement segmentée à partir d'une méthode de « clustering ». Des « features » permettant de décrire les A-scans sont choisis pour la mise en œuvre du « clustering ». Deuxièmement, une fois l'empreinte interne grossièrement segmentée, une estimation plus précise de la position de la jonction papillaire (couche de la peau formant l'empreinte interne) est effectuée grâce à des opérations de traitements d'images appliquées B-scan par B-scan. Dans cet article, l'empreinte interne n'est donc pas segmentée à partir de mesures de distances via la phase dans le domaine spectral. Par ailleurs, aucune fusion d'images n'est réalisée.

L'article Biometric Mapping of Fingertip Eccrine Glands With Optical Coherence Tomography IEEE Photonics Technology Letters Vol 22, No.22, November 15, 2010 divulgue un procédé visant à obtenir une cartographie des pores de sudation. Il n'y a aucune mention de mesures de distances, plus spécifiquement de mesures de distances via la phase dans le domaine spectral.

L'article Impact of Quality-Based Fusion Techniques for Video-Based Iris Recognition at a Distance Nadia Othman and Bernadette Dorrizi IEEE TRANSACTIONS on INFORMATION FORENSICS AND SECURITY, VOL 10, No. 8, AUGUST 2015 décrit un procédé de fusion mise en œuvre pour améliorer la qualité d'images biométriques d'iris provenant de flux video, et non d'empreintes digitales.

Les demandes de brevet US 2014/0241596 et US 2015/016127 divulguent des procédés visant à représenter les écoulements sanguins dans les réseaux vasculaires du doigt, afin de vérifier que c'est bien l'image d'un doigt vivant qui est acquise. L'information pertinente ici est une information de type Doppler, et il est proposé d'effectuer des différences de phase entre des A-scans (profils de réflectivité en profondeur) successifs. Ce traitement implique nécessairement que la phase en question soit une phase dans le domaine « spatial ». Il n'y a aucune mesure effectuée sur la phase dans le domaine spectral.

Résumé

Il existe un besoin pour améliorer la qualité des informations de la morphologie de surface externe ou interne des matériels biologiques provenant de dispositifs d'acquisition par tomographie optique cohérente, afin notamment d'extraire et d'identifier de façon efficace des empreintes digitales internes ainsi que des empreintes digitales externes en conditions difficiles.

L'invention vise à satisfaire ce besoin, et elle y parvient grâce à un procédé d'extraction de caractéristiques morphologiques d'un échantillon de matériel biologique selon la revendication 1.

L'image contenant des informations d'intensité et l'image contenant des informations de phase ne sont pas équivalentes en termes de contenu d'information. Même si leurs qualités sont comparables, les informations qu'elles contiennent sont complémentaires, et permettent de faciliter et d'optimiser l'extraction des caractéristiques morphologiques de l'échantillon à utiliser.

Le procédé selon l'invention peut ainsi être utilisé dans le domaine de la biométrie hautement sécurisée, visant la détection de fraude dans l'identification de personnes, en utilisant notamment l'empreinte digitale interne pour la comparer à l'empreinte externe, ou l'identification biométrique fiable en conditions difficiles, par exemple dans le cas de doigts humides ou sales, ou dans le cas d'une empreinte digitale externe plus ou moins effacée.

Dans le cas d'un doigt humide, dans l'image contenant les informations de phase, les maxima d'intensité rétrodiffusée sont toujours localisés au niveau de l'empreinte externe, et pas au niveau de la couche d'eau ou des gouttelettes. La récupération de la position de ces maxima d'intensité, grâce à la connaissance du temps de vol de la lumière, permet de reconstruire convenablement la structure 3D de l'empreinte externe. La mesure précise du temps de vol est avantageusement effectuée à partir de la connaissance de la phase du signal dans le domaine spectral. L'image en phase obtenue est ainsi de bien meilleure qualité, , assurant des performances plus robustes en identification biométrique que celle obtenues à partir des seules images en intensité, telles qu'obtenues par les capteurs biométriques connus ou bien avec les méthodes d'imagerie OCT de l'état de l'art.

Exploiter les informations de phase, correspondant au temps de vol de la lumière, permet de compenser la variabilité de l'intensité de diffusion de la lumière en jeu dans les propriétés de l'image, et notamment le fait qu'elles sont sensibles à l'angle d'incidence du faisceau lumineux avec la normale de la surface de l'échantillon à étudier.

Image en phase

Le deuxième pic de l'intensité d'un profil « *A-scan* », numéroté 2 à la figure 4, est attribué à l'empreinte digitale interne, ou à l'interface surcouche/doigt dans le cas d'une fraude. Il reflète les fortes inhomogénéités de la peau au niveau du derme papillaire, une couche de la peau située entre le derme et l'épiderme, correspondant à un changement d'organisation cellulaire, visible à la figure 7. Ainsi, de la même manière que précédemment décrit, il est avantageux de reconstruire une représentation 3D de l'empreinte interne en récupérant la position du deuxième pic de plus grande réflectivité pour chaque profil « *A-scan* » du volume tomographique.

Un profil de réflectivité de la lumière en profondeur étant établi à partir du signal représentatif de l'échantillon délivré par le système d'acquisition, ce profil de réflectivité présentant plusieurs pics de réflectivité maximale, on peut déterminer, afin de former l'image contenant des informations de phase, la position d'un pic de réflectivité maximale dudit profil de réflectivité, choisi selon le type de données à extraire. Le pic d'intérêt pour l'empreinte externe correspond de préférence au premier pic de réflectivité maximale du profil de réflectivité, et le pic d'intérêt pour l'empreinte interne correspond de préférence au deuxième pic. On peut obtenir au final des surfaces 3D associées aux empreintes externe ou interne, suivant le pic considéré.

Une fois la position du pic d'intérêt déterminée, un filtrage spatial du signal peut être effectué, notamment un filtrage passe-bande de l'interférogramme dans le domaine spatial, consistant au moins à retenir le signal interférométrique contenu dans une fenêtre centrée sur le pic d'intérêt et de largeur prédéfinie, notamment de l'ordre de grandeur de la résolution axiale du système d'acquisition OCT. Une transformation est alors avantageusement appliquée à ce signal afin d'obtenir des informations spectrales, notamment des informations spectrales en intensité et en phase concernant la diffusion enregistrée à l'interface air/doigt dans le cas d'une empreinte digitale externe ou à l'interface épiderme/derme dans le cas d'une empreinte digitale interne, la transformation étant notamment une transformée de Hilbert pour obtenir le signal interférométrique complexe. Afin d'obtenir les informations de phase recherchées, la pente de la phase est avantageusement calculée par une régression linéaire de la dépendance spectrale de la phase, obtenue à partir des informations spectrales obtenues par transformation du signal spatialement filtré.

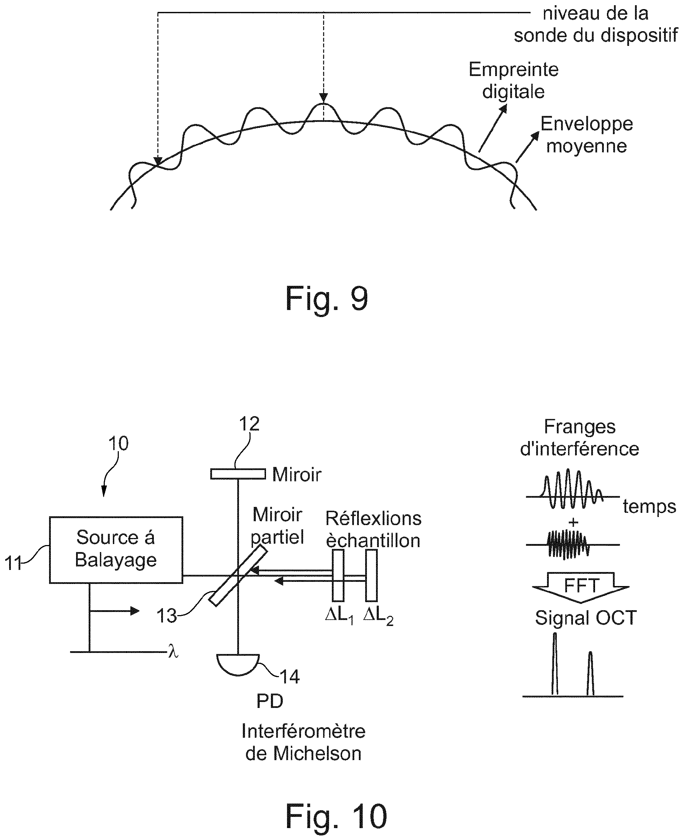

Pour une empreinte digitale, la référence utilisée pour mesurer les informations de phase est de préférence l'enveloppe moyenne de la surface du doigt. Cette enveloppe moyenne correspond à la surface enveloppe du doigt sans les sillons, comme représenté à la figure 9. Une surface 3D peut être codée comme une image topographique $S(x, y)$, où à chaque $(x, y)$ est associé une valeur de profondeur, ou de préférence ici, une valeur de temps de vol ou de phase. L'enveloppe moyenne, appelée $Em(x, y)$, est alors obtenue en appliquant un filtre moyenneur, notamment un filtre passe-bas 2D, sur l'image topographique $S(x, y)$. Les sillons étant de fréquences spatiales plus élevées, ceux-ci sont avantageusement supprimés lors de l'opération de filtrage.

Pour l'empreinte interne, l'enveloppe moyenne correspond à une surface interne du doigt, localisée au niveau de l'empreinte interne, obtenue en filtrant l'image tomographique associée à la surface 3D de l'empreinte interne.

Une image de textures 2D $P(x, y)$, dite image en phase, peut être obtenue en faisant la soustraction entre $S(x)$ et $Em(x, y)$ : $P(x,y) = S(x, y) - Em(x, y)$. De cette manière, la référence des mesures de temps de vol ou de phase n'est plus prise au niveau de la sonde du capteur mais au niveau de l'enveloppe moyenne. Par conséquent, l'image résultante représente avantageusement non pas les valeurs de phase $\Phi$, mais plutôt leurs variations $\Delta\Phi$, ce qui permet d'avoir une image de texture plus contrastée.

Le contraste de cette image de texture peut être encore amélioré en appliquant une égalisation d'histogramme adaptative puis un ajustement de contraste utilisant une fonction sigmoïde, dont le milieu est déterminé par la méthode d'Otsu, consistant à considérer que l'image à binariser ne contient que deux classes de pixels, le premier plan et l'arrière-plan, et à calculer le seuil optimal qui sépare ces deux classes afin que leur variance intra-classe soit minimale.

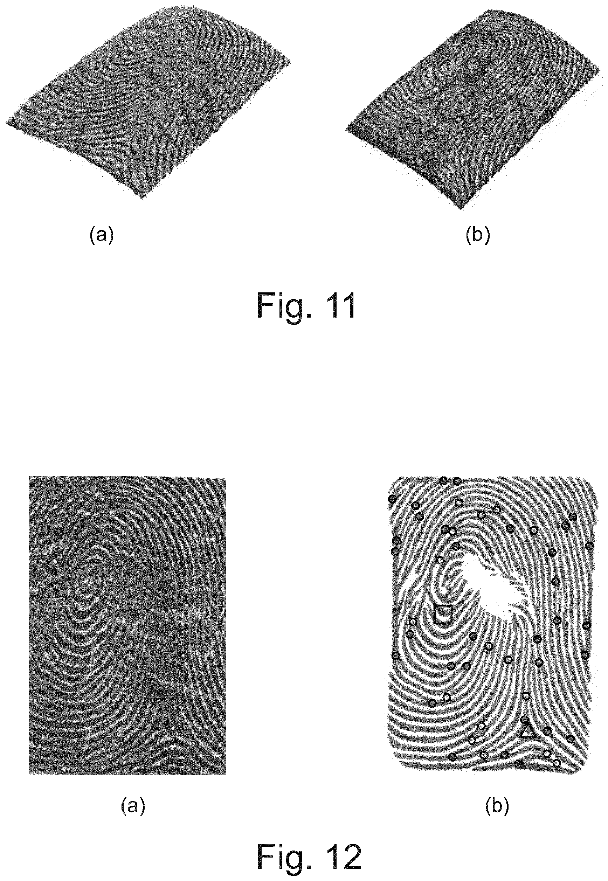

De la même manière que pour l'image $I(x, y)$, l'image de textures $P(x, y)$ peut être projetée sur la surface 3D de l'empreinte interne représentée à la figure 11(a).

L'invention permet de détecter les fraudes par utilisation de surcouches, en comparant l'empreinte digitale associée au premier pic de réflectivité maximale à celle associée au deuxième pic. Si ces empreintes sont différentes, il y a fraude.

L'invention a encore pour objet, selon un autre de ses aspects, un procédé pour générer une image comportant une information liée à la position d'une interface entre deux milieux, notamment l'air et la peau ou le derme et l'épiderme, à partir d'un interférogramme d'une empreinte, digitale ou palmaire, interne ou externe obtenu par SW-OCT dans le domaine spectral, comportant les étapes consistant à :

- Appliquer à l'interférogramme une transformation, notamment de Fourrier, pour générer un réflectogramme dans le domaine temporel,
- estimer à partir de ce réflectogramme une position spatiale de l'interface correspondant à l'image de l'empreinte interne ou externe que l'on cherche à imager.

La position spatiale peut être estimée à partir de l'enveloppe du signal du réflectogramme, en prenant comme position le maximum local de l'amplitude de l'enveloppe du signal au niveau d'un pic de réflectivité correspondant à l'interface recherchée.

De préférence, la position est déterminée en appliquant un filtrage passe bande pour isoler le pic de réflectivité correspondant à l'interface recherchée et en appliquant au réflectogramme ainsi filtré une transformation inverse, notamment une transformation de Fourrier inverse, pour générer un interférogramme filtré, dans le domaine spectral.

Cet interférogramme peut être utilisé pour déterminer la pente de la droite de régression linéaire de la phase spectrale $\phi(\upsilon)$ en fonction de la fréquence $\upsilon$, puis le temps de vol $\tau_0$ et la position $z_0$ de l'interface correspondant au pic de réflectivité par la formule

$$\tau_0 = 1/2\pi \, d\phi(\upsilon) \, / d \, \upsilon = z_0/c$$

Une surface 3D peut être générée à partir de la connaissance de la position $z0$ en chaque point x,y du pic de réflectivité considéré, et donc de l'interface concernée, Le premier pic peut donner la position de l'interface air/peau, correspondant à l'empreinte externe, et la deuxième celle de l'interface épiderme/derme correspondant à l'interface interne.

Un filtrage passe-bas 2D peut être appliqué à une telle surface pour obtenir une enveloppe moyenne Em(x,y) de la position de l'interface.

Cette enveloppe peut être prise comme référence pour générer par soustraction une image de texture en phase P(x,y).

Cette image de texture en phase peut être fusionnée avec une image de texture en intensité I(x,y) pour obtenir une image de texture de fusion, laquelle peut être ensuite projetée une surface 3D afin d'obtenir une surface 3D d'empreinte texturée aplatie.

On peut procéder ainsi pour obtenir des images d'empreintes digitales interne et externe.

Fusion des images en intensité et en phase

Dans un mode de réalisation préféré de l'invention, l'image contenant des informations d'intensité et l'image contenant des informations de phase sont fusionnées pour former une seule image.

Pour ce faire, la structure de chaque image contenant respectivement des informations d'intensité et des informations de phase est analysée afin d'établir pour chaque image une carte de confiance contenant une valeur de qualité pour chaque pixel, en fonction des pixels voisins. Les cartes de confiance des images se fondent notamment sur la présence d'un bon contraste et sur la qualité locale des sillons présents sur les images, dans le cas d'une empreinte digitale.

Chaque pixel de l'image de fusion *F* entre l'image *I* contenant des informations d'intensité et l'image *P* contenant des informations de phase est avantageusement issu d'une combinaison linéaire des valeurs des pixels correspondants des deux images, pondérées par les valeurs de qualité des cartes de confiance :

$$F(x,y) = \alpha_I(x,y) \times I(x,y) + \alpha_P(x,y) \times P(x,y),$$

avec par exemple $\alpha_I = \dfrac{C_I(x,y)}{Norm}$ et $\alpha_P = \dfrac{C_P(x,y)}{Norm}$, (x, y) les coordonnées d'un pixel, $C_I(x,y)$ la valeur de qualité du pixel (x, y) de l'image I, avec $0 < C_I(x,y) < 1$, $C_P(x,y)$ la valeur de qualité du pixel (x, y) de l'image P, avec $0 < C_p(x,y) <$

1, et *Norm* = $C_I(x,y)$ + $C_P(x,y)$.

Si *Norm* = 0, on fixe de préférence $\alpha_I$ = $\alpha_P$ = 0.5. Selon la formule de fusion utilisée, les valeurs $\alpha_I$ et $\alpha_P$ peuvent être exprimées différemment, l'invention n'est pas limitée à un calcul particulier pour les valeurs $\alpha_I$ et $\alpha_P$.

Dans une variante, l'image de fusion entre l'image contenant des informations d'intensité et l'image contenant des informations de phase est avantageusement formée en retenant, pour chaque pixel, le pixel de l'image ayant la valeur de qualité la plus élevée :

$$F(x,y) = \begin{cases} I(x,y)\, si\ C_I(x,y) > C_P(x,y) \\ P(x,y)\, si\ C_I(x,y) < C_P(x,y) \end{cases}.$$

L'image de fusion entre l'image contenant des informations d'intensité et l'image contenant des informations de phase est ainsi avantageusement formée pixel par pixel, en fonction du voisinage de chaque pixel, grâce aux cartes de confiance.

Dans le cas où l'image considérée est une empreinte digitale, la valeur de qualité d'un pixel, ($C_P(x,y)$ *ou* $C_I(x,y)$), peut être obtenue à partir de cartes de fiabilité des champs d'orientation des sillons de l'empreinte, ou « *orientation field reliability map* » en anglais, comme décrit dans les articles de J. Zhou et J. Gu, "A Model-based for the computation of fingerprint's orientation field", IEEE Transactions on Image Processing, vol. 13, no. 6, 2004, et de M. S. Khalil, "Deducting fingerprint singular points using orientation field reliability", First conference on robot, vision and signal processing, pp. 234-286, 2011. Les champs d'orientation des sillons représentent la direction des sillons pour chaque position dans l'empreinte. Ils sont calculés pour chaque pixel de l'image d'empreinte digitale, en fonction de leur voisinage. L'utilisation de tels champs d'orientation en biométrie des empreintes digitales est connue, par exemple dans des méthodes d'amélioration d'images d'empreintes digitales telles que celle décrite dans l'article de L. Hong et al. "Fingerprint image enhancement: algorithm and performance evaluation", IEEE Transactions on Pattern Analysis and Machine Intelligence, vol.20, no.8, 1998. Les cartes de fiabilité des champs d'orientation permettent d'évaluer la validité et la fiabilité de l'estimation de l'orientation des sillons.

Une région d'image d'empreinte digitale de faible qualité peut être caractérisée par le fait que la texture des sillons n'est pas apparente, la structure périodique caractéristique des sillons n'y étant pas retrouvée. Dans de telles régions, l'estimation de l'orientation est mauvaise car il n'y a pas d'orientation prépondérante. Par conséquent, la valeur de la fiabilité est faible. Inversement, dans des régions d'empreintes très structurées, la présence d'une direction particulière peut être estimée de manière fiable. La valeur de la fiabilité pour ces régions est élevée.

Comme expliqué dans les articles de C. Sousedik et al. "Volumetric Fingerprint Data Analysis using Optical Coherence Tomography", BIOSIG Conference, 2013, pp. 1-6, et de C. Sousedik et C. Bush, " Quality of fingerprint scans captured using Optical Coherence Tomography", IJCB Conference, 2014, pp. 1-8, la structure de l'empreinte interne peut être assez inhomogène, contrairement à celle de l'empreinte externe, qui est bien continue, conduisant à une ambigüité sur la position du deuxième pic de réflectivité maximale. La structure de l'empreinte interne peut également varier grandement d'un individu à l'autre. Détecter la position de l'empreinte interne par des mesures de temps de vol peut être délicat, dans la mesure où l'interface n'est pas obligatoirement bien définie.

En outre, la rétrodiffusion de la lumière dans la peau fait intervenir des phénomènes physiques complexes et difficiles à prédire, liés aux interférences entre les ondes multiples rétrodiffusées par les structures biologiques. Il n'est pas évident que, dans une empreinte digitale, les sommets des sillons correspondent à des maxima de réflectivité et les creux à des minima, ou vice versa.

La fusion des images en phase et en intensité permet de tirer le meilleur profit des informations disponibles sur l'ensemble des deux images, et ainsi d'améliorer de façon importante la qualité de l'image finale obtenue sur la surface recherchée. Par exemple dans le secteur de la biométrie, il en découle une amélioration importante des performances d'identification basée sur l'empreinte sous-cutanée, en utilisant les algorithmes d'identification biométriques connus.

Précision de localisation

La précision de localisation des pics de réflectivité maximale conditionne en partie la qualité des images *3D* et *2D* en phase. La précision de localisation, différente de la résolution axiale, est une notion peu connue de l'art antérieur, et peu exploitée dans les applications biomédicales.

La résolution axiale correspond à la distance minimale nécessaire entre deux centres de diffusion afin de pouvoir les distinguer correctement, et est seulement dépendante de la largeur spectrale de la source lumineuse. Elle peut être mesurée à partir de la largeur à mi-hauteur d'un pic associé à un unique centre de diffusion, par exemple le premier pic numéroté 1.

La précision de localisation est avantageusement liée à l'erreur de localisation du maxima de l'enveloppe des différents profils « *A-scan* ». Afin d'évaluer la précision de localisation, une étude statistique est réalisée, consistant à simuler le pic associé à un unique centre de diffusion, dont la position est fixée lors de la simulation, en prenant en compte les différentes contributions de bruit du photo-détecteur du système d'acquisition, principalement le bruit thermique et le bruit de grenaille qui ont des distributions assimilables à un bruit blanc. Ce bruit peut avoir un impact plus ou moins important, selon sa puissance, sur la position mesurée du pic maximum. L'erreur faite sur la position peut être évaluée par la différence entre la position du maximum du profil « *A-scan* » bruité simulé et celle du profil « *A-scan* » de référence utilisé, connue au préalable. On définit alors la précision de localisation du système d'acquisition par l'écart type de cette erreur de localisation. L'écart type est avantageusement obtenu à partir d'un grand nombre de tirages aléatoires de profils « *A-scan* » bruités.

Dispositif

Selon un autre de ses aspects, l'invention concerne un dispositif d'extraction de caractéristiques morphologiques d'un échantillon de matériel biologique selon la revendication 11.

Dans un mode de réalisation préféré de l'invention, le dispositif est configuré en outre pour fusionner l'image contenant des informations d'intensité et l'image contenant des informations de phase afin de former une seule image.

Les caractéristiques décrites précédemment pour le procédé selon l'invention s'appliquent au dispositif.

Le champ de vue du dispositif, correspondant à l'étendue spatiale maximale dans le plan X-Y pouvant être enregistrée, peut être étendu, atteignant par exemple 2 mm par 2 mm, soit 4 mm$^2$, mieux 2 cm par 2 cm, soit 4cm$^2$. Cela permet d'obtenir un nombre important de minuties, dans le cas de l'extraction d'une empreinte digitale.

L'invention pourra être mieux comprise à la lecture de la description détaillée qui va suivre, d'exemples non limitatifs de mise en œuvre de celle-ci, et à l'examen du dessin annexé, sur lequel :

- la figure 1, précédemment décrite, représente une image volumique obtenue par un système d'acquisition par tomographie optique cohérente sur un doigt,
- les figures 2(a) et 2(b), précédemment décrites, représentent, respectivement, l'image en intensité et l'image traitée obtenues, selon l'art antérieur, à partir du volume de la figure 1,
- les figures 3(a) et 3(b), précédemment décrites, représentent, respectivement, l'acquisition d'une empreinte digitale par tomographie et le profil « A-*scan* » obtenu en fonction du temps de vol de la lumière,
- la figure 4, précédemment décrite, représente l'intensité d'un profil « *A-scan* » en fonction de la profondeur,
- la figure 5, précédemment décrite, illustre la présence de gouttes d'eau à la surface d'un doigt,
- la figure 6, précédemment décrite, représente l'image en intensité du doigt humide de la figure 5, obtenue par OCT selon l'art antérieur,
- la figure 7, précédemment décrite, représente la coupe transversale d'un volume tomographique obtenu selon l'art antérieur,
- la figure 8, précédemment décrite, représente l'empreinte digitale interne 3D obtenue à partir du volume de la figure 7 obtenu selon une méthode l'art antérieur,
- la figure 9, précédemment décrite, illustre l'enveloppe moyenne de la surface d'un doigt,
- la figure 10 représente un dispositif OCT selon l'invention,
- la figure 11(a) représente l'image en phase et la figure 11(b) représente l'image en intensité de l'empreinte interne, projetées sur la surface 3D correspondante, obtenues par la mise en œuvre du procédé selon l'invention, à partir du volume tomographique de la figure 1,
- les figures 12(a) et 12(b) représentent, respectivement, l'image en phase et l'image traitée de l'empreinte interne, obtenues, selon l'invention, à partir du volume tomographique de la figure 1,
- la figure 13 illustre une comparaison entre deux images en phase obtenues selon l'invention,
- les figures 14(a) et 14(b) représentent, respectivement, l'image fusionnée des informations en phase et en intensité, et l'image traitée, obtenues selon l'invention,
- la figure 15 représente des empreintes digitales internes, les minuties associées, et la carte de fiabilité de l'orientation des sillons pour les images en phase, en intensité et pour la fusion des deux, obtenues selon l'invention,
- la figure 16 est un graphe présentant des courbes de performance obtenues par la mise en œuvre du procédé selon l'invention,
- la figure 17 est un graphe représentant les densités de probabilité de scores client et de scores imposteur, utilisant une base de données d'images d'empreintes internes extraites selon l'invention,
- la figure 18 représente une image en intensité d'une empreinte digitale dans le cas d'un doigt humidifié,
- les figures 19(b) et 19(a) représentent, respectivement, une image après fusion d'une empreinte digitale dans le cas du doigt humidifié de la figure 17, obtenue selon l'invention, et l'image en phase correspondante,

- la figure 20 est un graphe représentant l'erreur de localisation selon l'invention en fonction du rapport signal à bruit et de la résolution axiale,
- la figure 21 est un graphe présentant des courbes de performance comparatives,
- la figure 22 illustre une comparaison d'images obtenues à partir d'un doigt humide, avec des capteurs selon l'art antérieur et selon l'invention, et
- les figures 23 à 26 illustrent différentes étapes d'un exemple de procédé selon l'invention.

Un dispositif OCT 10 permettant de mettre en œuvre l'invention est représenté à la figure 10. Ce dispositif 10 comporte une source à balayage 11 configurée pour balayer l'échantillon à différentes profondeurs, un miroir 12, un miroir partiel 13, et un interféromètre de Michelson 14. Chaque balayage de longueur d'onde, ou « *A-scan* » en anglais, produit des franges d'interférence à partir des réflexions de l'échantillon à différentes profondeurs.

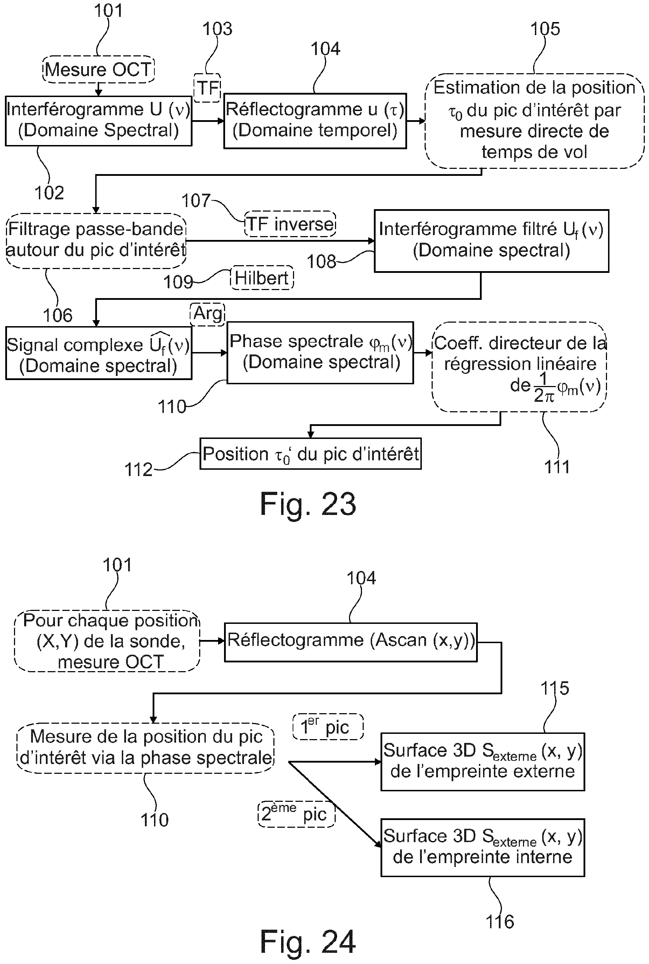

On va maintenant décrire en référence aux figures 23 à 25 un exemple de procédé selon l'invention, pour chaque position x,y de la sonde.

Comme illustré à la figure 23, pour obtenir les images en phase P(x,y) on peut partir d'un interférogramme 102 dans le domaine spectral, à partir d'une mesure SW-OCT (étape 101).

Par transformée de Fourier 103 on obtient un réflectogramme 104 dans le domaine temporel, qui permet (étape 105) une estimation de la position du pic d'intérêt (interface air/doigt, épiderme/derme, ....) par mesure directe du temps de vol, à l'aide de l'enveloppe de chaque A-scan.

On peut faire subir à ce réflectogramme 104 un filtrage passe-bande dans le domaine spatial autour du pic d'intérêt (étape 106), ce qui isole ce pic, puis obtenir par une transformée de Fourier inverse 107 un interférogramme filtré dans le domaine spectral.

Une transformée de Hilbert 109 peut permettre de récupérer un signal complexe dans le domaine spectral, dont l'argument fournit la phase spectrale 110 et un traitement statistique 111 par régression linéaire le coefficient directeur $d\phi(\upsilon)/d\upsilon$ de la pente, et donc le temps de vol 102 du pic d'intérêt, c'est-à-dire sa position spatiale.

L'image générée à partir de l'information de phase spectrale consiste dans cet exemple en la représentation en niveau de gris du temps de vol $\tau$(x,y). Dans cette représentation, la résolution spatiale de la mesure n'est pas dégradée. La variation du temps de vol suivant les axes x et y permet d'accéder aux caractéristiques morphologiques de l'empreinte.

Une telle image se distingue du mode d'imagerie en intensité proposé dans l'article de Bossen et al cité plus haut. En effet, plutôt que de représenter le temps de vol $\tau$(x,y), Bossen propose de représenter I(x,y) où I(x,y) représente l'intensité moyenne de l'enveloppe du A-scan aux alentours du pic d'intérêt. La zone de moyennage spatial considérée, typiquement entre $100\mu$m-$900\mu$m, est bien plus large que la résolution spatiale de l'instrument. Dans cette représentation, c'est la variation de l'intensité I suivant les axes x et y qui permet d'accéder aux caractéristiques morphologiques de l'empreinte.

La figure 24 illustre la possibilité pour chaque mesure 101 en x,y et réflectogramme A-scan associé 104 d'appliquer le procédé décrit en référence à la figure 23 pour mesurer précisément la position 110 du pic d'intérêt via la connaissance de la phase spectrale $\phi(\upsilon)$, afin d'obtenir des images de surface 3D de l'empreinte externe 115 et de l'empreinte interne 116.

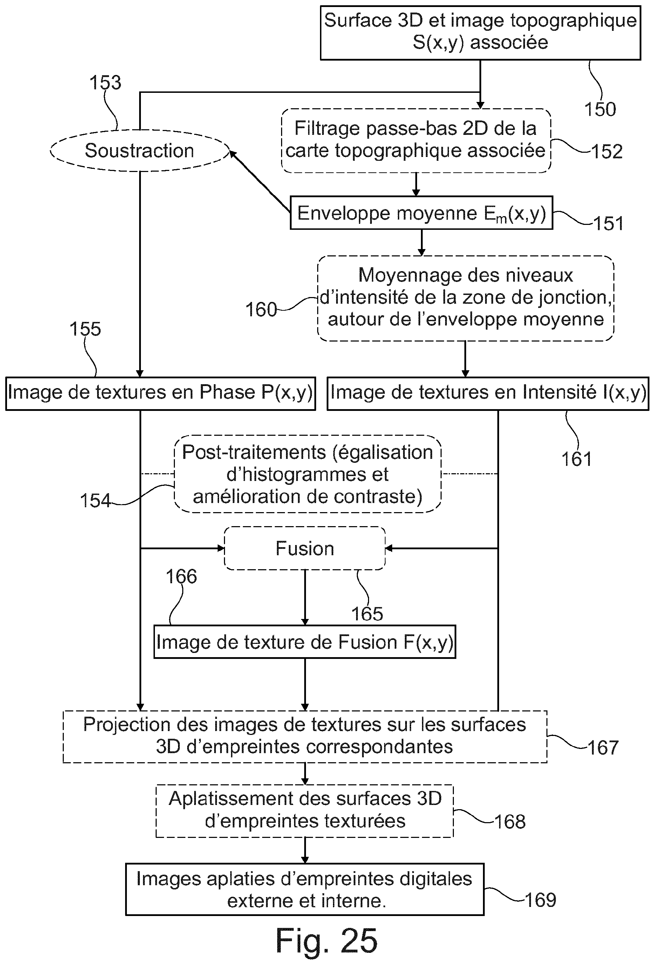

On va maintenant décrire en référence à la figure 25 un exemple de procédé de fusion des images en phase et en intensité, après extraction par un processus 140 tel que décrit par exemple en référence aux figures 23 et 24 des surfaces 3D des empreintes.

Dans le cas où l'échantillon est une empreinte digitale, la référence utilisée pour mesurer les informations de phase est de préférence l'enveloppe moyenne de la surface du doigt. Cette enveloppe moyenne correspond à la surface enveloppe du doigt sans les sillons. Une surface 3D peut être codée comme une image topographique S(x, y) 150, où à chaque (x, y) est associée une valeur de temps de vol ou de phase. L'enveloppe moyenne 151, appelée Em(x, y), est alors obtenue en appliquant un filtre moyenneur 152 , notamment un filtre passe-bas 2D, sur l'image topographique S(x, y). Les sillons étant de fréquences spatiales plus élevées, ceux-ci sont supprimés lors de l'opération de filtrage.

Une image de textures 2D P(x, y), dite image en phase, peut être obtenue en faisant la soustraction (étape 153) entre S(x) et $E_m$(x, y) : $P(x, y) = S(x, y) - Em(x,y)$. De cette manière, la référence des mesures de temps de vol ou de phase n'est plus prise au niveau de la sonde du capteur mais au niveau de l'enveloppe moyenne. Par conséquent, l'image résultante 155 représente avantageusement non pas les valeurs de phase spectrale $\Phi_m$, mais plutôt leurs variations $\Delta\Phi$, ce qui permet d'avoir une image de texture plus contrastée.

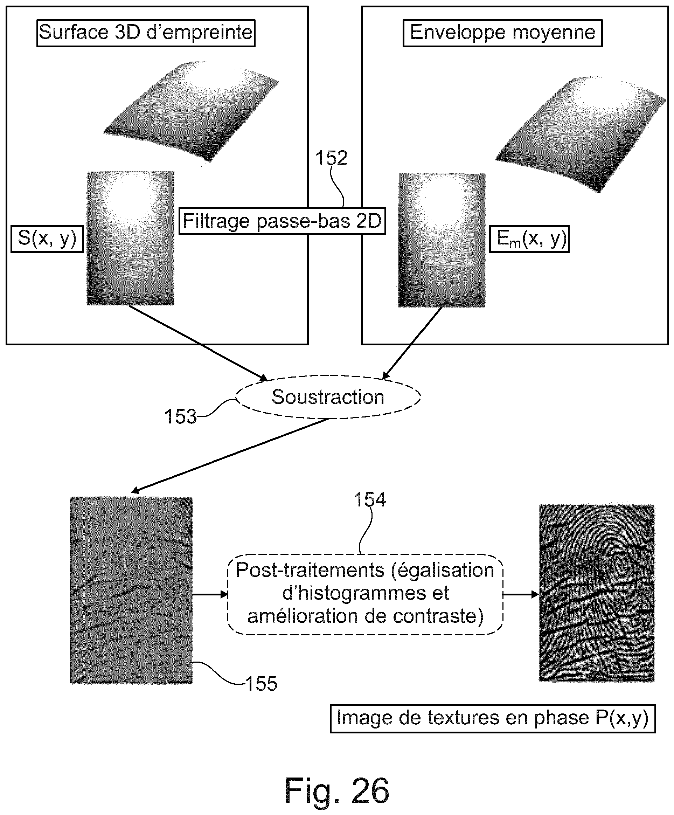

La figure 26 illustre un exemple de résultat obtenu en effectuant ces opérations.

Le contraste de cette image de texture peut être encore amélioré en appliquant une égalisation d'histogramme adaptative (étape 154) puis un ajustement de contraste utilisant une fonction sigmoïde, dont le milieu est déterminé par la méthode d'Otsu, consistant à considérer que l'image à binariser ne contient que deux classes de pixels, le premier plan et l'arrière-plan, et à calculer le seuil optimal qui sépare ces deux classes afin que leur variance intra-classe soit minimale.

La connaissance de l'enveloppe moyenne permet également de moyenner les niveaux d'intensité (étape 160) pour obtenir une image de texture en intensité 161, laquelle peut subir également un traitement d'ajustement du contraste (étape 154).

Les images en phase P(x,y) et en intensité I(x,y) peuvent être fusionnées à l'étape 165 pour obtenir une image de texture de fusion 166. Ces images de texture peuvent être projetées (étape 167) sur les surfaces 3D correspondantes.

Un aplatissement (étape 168) des surfaces 3D ainsi texturées peut être réalisé pour obtenir des images d'empreintes interne et externe aplaties 169.

Une image *3D* en phase de l'empreinte interne obtenue, selon l'invention, à partir du volume tomographique de la figure 1, est représentée à la figure 11(a).

L'image en intensité de la même empreinte interne, représentée à la figure 11(b), présente des zones inexploitables à très faible contraste. Ces zones sont aléatoires car elles dépendent entre autres des propriétés de diffusion locales du tissu biologique mais aussi de l'angle d'incidence de la sonde du système d'acquisition par tomographie optique cohérente, notamment dans le cas d'une mesure sans contact où la mesure est non reproductible.

La figure 12(a) représente une image en phase brute, la figure 12(b) représentant l'image correspondante délivrée à la sortie du « *matcher* ». Ces images sont à comparer aux images en intensité présentées à la figure 2, précédemment décrite. Les positions des zones inexploitables de l'image de la figure 12(b) sont différentes de celle de la figure 2(b). Ainsi, utiliser à la fois les caractéristiques extraites de l'image en intensité et celles de l'image en phase permet d'améliorer l'identification de la personne correspondant à cette empreinte digitale.

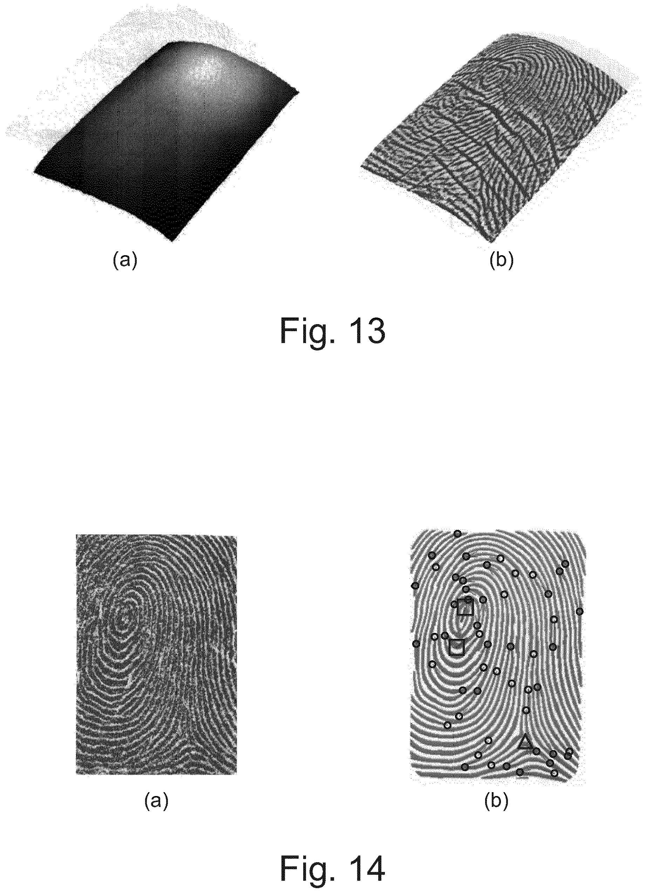

La figure 13(a) représente une image *3D* de l'empreinte externe sur laquelle a été projetée l'information de la phase, obtenue à partir du volume tomographique de la figure 1, selon l'invention. Les fortes valeurs, représentées en blanc, correspondent à un temps de vol court entre la sonde du système d'acquisition OCT et la surface de l'empreinte, et les faibles valeurs d'intensité, représentées en noir, correspondent à un temps de vol plus long. Cet exemple ne permet pas d'obtenir directement des images d'empreintes de bonne qualité, dans la mesure où l'on ne peut discerner convenablement les sillons. Cela est dû au fait que la référence pour mesurer le temps de vol, c'est-à-dire la sonde du système d'acquisition OCT, n'est pas située à une distance égale pour tous les points de la surface du doigt. Afin d'obtenir un meilleur contraste, comme décrit précédemment, l'enveloppe moyenne de la surface du doigt est prise comme référence pour le temps de vol. Comme visible à la figure 13(b), représentant l'empreinte 3D sur laquelle a été projetée l'information en delta-phase, c'est-à-dire les variations de la phase, correspondant à l'information pertinente pour avoir des images d'empreintes bien contrastées, les sillons sont dans ce cas bien visibles.

Comme décrit précédemment, l'image contenant des informations d'intensité et l'image contenant des informations de phase sont fusionnées pour former une seule image, en utilisant les cartes de confiance de chaque image délivrant des valeurs de qualité pixel par pixel. Une image formée par la fusion de l'image en intensité de la figure 2(a) et de l'image en phase de la figure 12(a) est visible à la figure 14(a), l'image correspondant à la sortie du « *matcher* » étant représentée à la figure 14(b). Grâce à la fusion, l'image résultante est de bien meilleure qualité, les zones inexploitables ayant quasiment disparu.

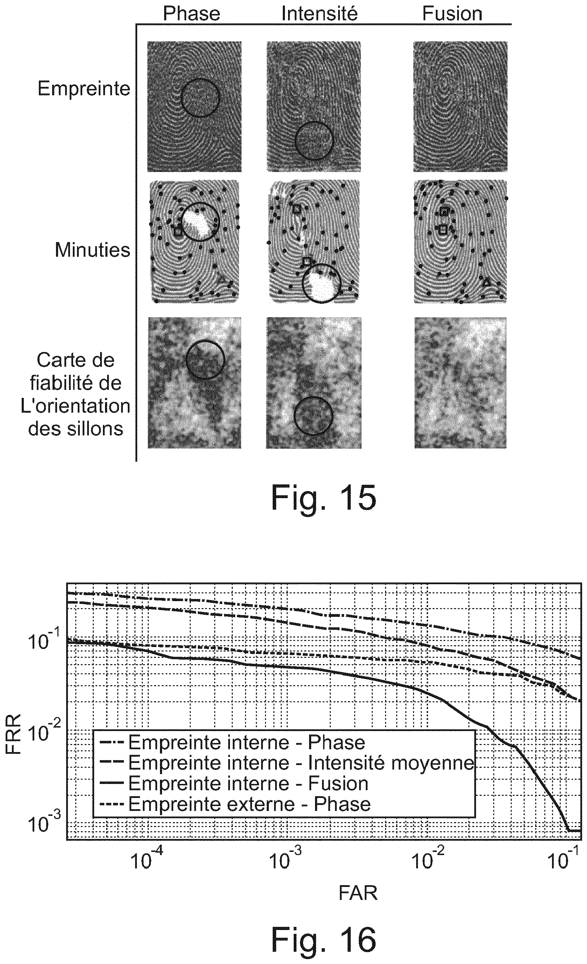

La figure 15 représente des empreintes digitales internes, les minuties associées, et la carte de fiabilité de l'orientation des sillons pour les images en phase, en intensité et pour la fusion des deux. Les images complémentaires ont été considérées afin d'utiliser la représentation connue des empreintes digitales. Les images sur la première ligne correspondent aux images d'empreinte interne aplatie, dans les trois représentations. Les images de la deuxième ligne représentent les mêmes images après des étapes de prétraitements et de binarisation, le logiciel Verifinger, développé par Neurotechnology, ayant été utilisé dans l'exemple décrit. Sur ces images, les minuties extraites à partir de l'image binarisée, représentées par les points noirs, exploitées par les « *matchers* », sont utilisées pour l'étape d'identification par la mise en correspondance des minuties de deux images d'empreinte digitale. Pour les deux représentations en phase et en intensité, la qualité d'image est médiocre pour certaines régions, comme représenté par des cercles noirs. Pour de telles régions, les sillons des empreintes digitales ne sont pas visibles. Par conséquent, la qualité de ces régions n'est pas suffisante pour assurer une détection de minuties correcte, comme illustré par les trous blancs dans les images binarisées. Dans les représentations des cartes de fiabilité des champs d'orientations des sillons, les pixels sombres correspondent aux valeurs faibles de fiabilité tandis que les pixels clairs correspondent aux valeurs élevées. Dans les représentations en intensité et en phase, de faibles valeurs de fiabilité sont associées aux zones de mauvaise qualité. Il est à noter que, de préférence et dans l'exemple décrit, les régions problématiques ne sont pas localisées au même endroit dans les deux représentations.

Comme visible sur la dernière colonne de la figure 15, l'image d'empreinte digitale interne après la fusion des images en intensité et en phase est de bien meilleure qualité, cette image ayant été reconstruite en choisissant les meilleures régions des deux représentations. La structure des sillons est mieux préservée sur l'ensemble de l'image. Les régions avec des trous dans l'image binarisée ont disparu, ce qui amène à une détection de minuties plus robuste. La carte de fiabilité pour l'image après fusion illustre bien l'amélioration de la qualité globale de l'image, les zones claires étant plus nombreuses et plus étendues.

La figure 16 représente une comparaison des performances obtenues pour des résultats provenant des différentes représentations, sur une base comprenant une centaine de doigts, en termes de taux de fausse acceptation FAR en fonction du taux de faux rejets FRR. Ces courbes de compromis d'erreur de détection (DET pour « *detection error tradeoff* » en anglais), donnant le taux de fausse acceptation en fonction du taux de faux rejets, sont connues pour évaluer les performances des systèmes biométriques. Plus ces courbes sont situées vers le bas, meilleures sont les performances, un taux de faux rejets minimum étant recherché pour un taux de fausse acceptation donné. La courbe en petits pointillés correspond à la courbe de référence, obtenue avec une image en phase de l'empreinte externe, cette empreinte étant par nature aisément accessible aux différents capteurs.

La courbe en pointillés larges et la courbe en pointillés discontinus correspondent respectivement aux courbes des empreintes internes extraites par les images en intensité et en phase, et sont environ au même niveau. Pour un taux de fausse acceptation de $10^{-3}$ par exemple, le taux de faux rejets est dégradé d'un facteur 2-3 par rapport au taux de faux rejets associé à la courbe de référence. Ce résultat témoigne de la difficulté d'accéder à l'empreinte interne. La courbe en traits pleins est calculée à partir des images après fusion. Pour ce même taux de fausse acceptation, le taux de faux rejets est diminué d'un facteur d'environ 3-4 par rapport à celui associé aux courbes correspondant aux images d'empreinte interne en phase et en intensité. Dans un autre exemple, pour un taux de fausse acceptation de 0.01%, le taux de faux rejets est d'environ 7% pour les images après fusion, contre 26% pour les images en phase et 20% pour les images en intensité. Pour un taux de fausse acceptation de 0.1%, le taux de faux rejets est d'environ 4% pour les images après fusion, contre 20% pour les images en phase et 14% pour les images en intensité. Il est à noter en outre que les performances sont meilleures avec les images d'empreinte interne après fusion qu'avec les images d'empreinte externe en phase, les empreintes internes étant mieux préservées que les empreintes externes.

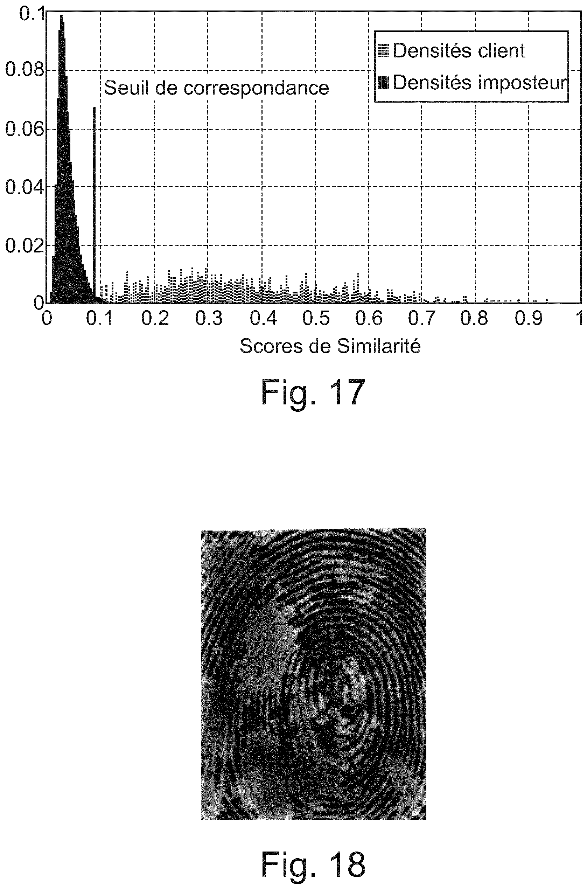

Des densités de probabilité de scores client et imposteur sont représentées à la figure 17, obtenues sur une base de données d'images d'empreintes internes extraites selon l'invention, comportant 102 doigts différents issus de 15 individus, chaque doigt ayant été acquis 4 fois. Les images d'empreintes internes dans les trois représentations, intensité, phase et après fusion, ont été extraites des volumes tomographiques. Pour les tests de vérification, chaque image d'empreinte interne a été comparée avec toutes les autres images de la base, conduisant à un total de 166056 comparaisons d'empreintes. La comparaison de deux images provenant d'un même doigt est appelée correspondance client, ou « *genuine matching* » en anglais, et la comparaison de deux images issues de doigts différents est appelé correspondance imposteur, ou « *impostor matching* ». Les scores de similarité sont calculés avec le logiciel NBIS (NIST Biometric Image Software). Dans cet exemple, l'algorithme MINDTCT permet d'extraire les minuties d'une image d'empreinte et le « *matcher* » BOZORTH3 retourne le score de similarité entre deux images. Deux densités de probabilité de scores, la densité client et la densité imposteur, sont obtenues, la capacité à discerner ces densités permettant de quantifier les performances d'un système biométrique. La décision finale est prise en comparant le score de similarité obtenu à un seuil, choisi en fonction des densités de score et des performances souhaitées. Comme les densités client et imposteur se recouvrent, des erreurs de faux rejets ou de fausses acceptations sont faites lors de la prise de décision. Les performances en vérification sont finalement évaluées à l'aide des courbes de performance obtenues en faisant varier le seuil de correspondance.

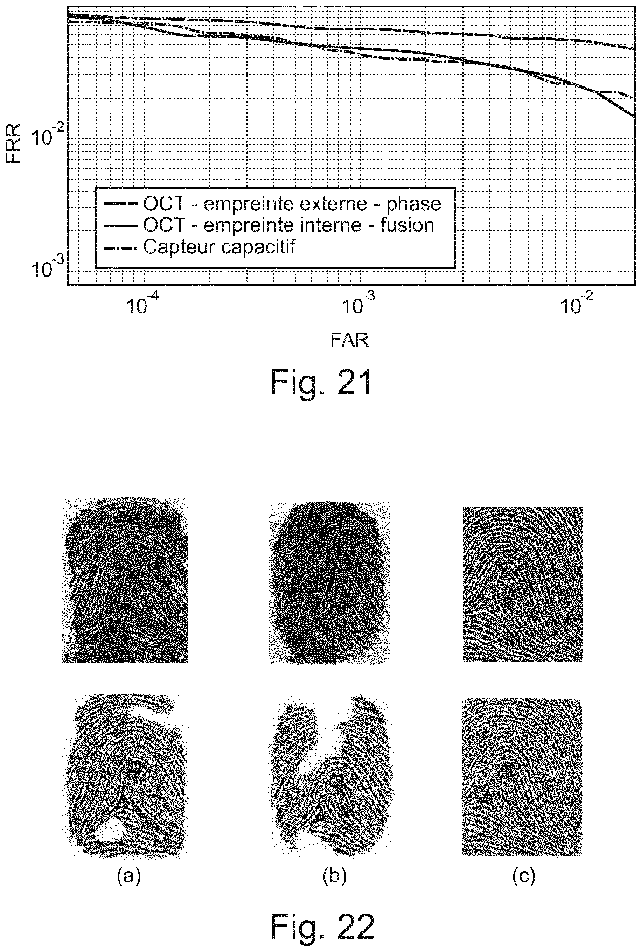

Les performances obtenues démontrent que l'empreinte interne permet une identification des personnes avec des performances comparables à celles obtenues par les lecteurs biométriques connus sur l'empreinte externe d'un doigt sec. L'identification des personnes présentant des doigts sales ou humides est également plus efficace que ce qui est possible en utilisant les systèmes biométriques connus. La figure 21 présente une comparaison des performances obtenues par l'utilisation de l'empreinte interne extraite par fusion selon l'invention à celles obtenues par l'utilisation de l'empreinte externe extraite par les capteurs selon l'art antérieur, par exemple un capteur 2D capacitif. Des FRR similaires sont obtenus pour un FAR fixe.

Par extension, dans le cas de doigts humides, les performances obtenues par utilisation de l'empreinte interne extraite par fusion selon l'invention sont meilleures que celles obtenues par les capteurs selon l'art antérieur, par exemple un capteur 2D capacitif. En effet, les performances du capteur 2D capacitif dans le cas humide sont forcément moins bonnes que celles présentées dans le cas normal, illustrées par la courbe en pointillés de la figure 21.

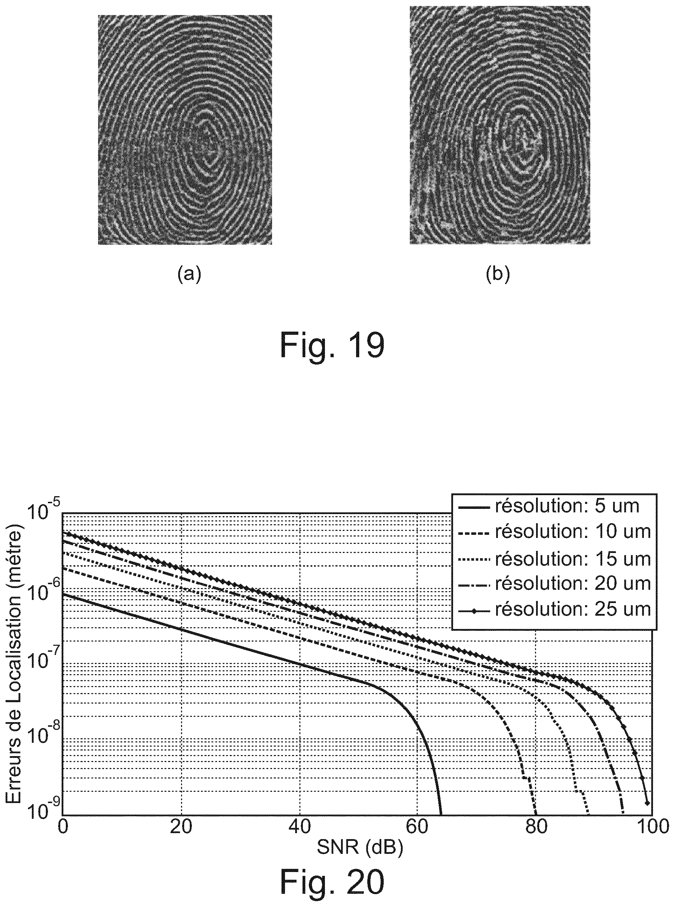

Les figures 18 et 19 montrent des empreintes digitales obtenues dans le cas de doigts humides. Comme visible à la figure 18, l'image en intensité présente des zones très peu contrastées au niveau des zones humides. Les images correspondantes en phase et après fusion, obtenues selon l'invention, sont représentées respectivement aux figures 19(a) et 19(b). L'image en phase est de meilleure qualité que l'image en intensité, ne présentant presque pas de défauts pouvant nuire à l'identification de l'empreinte et directement exploitable, et l'image après fusion est aussi de très bonne qualité,.

Les figures 22(a) et 22(b) représentent les images d'empreintes digitales d'un doigt humide pour deux capteurs 2D connus, respectivement un capteur optique et un capteur capacitif. Des tâches noires dues à l'humidité excessive du doigt sont visibles dans les images. Ces tâches dégradent considérablement la qualité des images, et diminuent par conséquent les performances en authentification. Les images binarisées correspondantes montrent que les zones tâchées n'ont pas été reconnues dans l'empreinte digitale. En comparaison, l'image en phase obtenue selon l'invention, représentée à la figure 22(c), est de bien meilleure qualité.

La figure 20 représente l'écart type de l'erreur de localisation en fonction du rapport signal à bruit SNR, défini

**EP 3 147 822 B1**

comme le rapport entre le niveau d'intensité du pic et celui du bruit de fond, comme décrit précédemment en référence à la figure 4, pour différentes résolutions axiales, de 5 μm à 25 μm. Pour un rapport signal à bruit de 50 dB, correspondant à une valeur typique pour une rétrodiffusion à l'interface air/peau, l'erreur de localisation est estimée entre 60 nm et 350 nm. L'erreur de localisation est bien en dessous de la résolution axiale du système d'acquisition, évaluée à environ 10 μm dans l'exemple considéré. La précision de localisation est également bien en dessous de l'ordre de grandeur de la longueur d'onde de la source lumineuse utilisée, environ égale à 1300 nm.

En considérant, selon le principe d'ergodicité, que les statistiques d'ensemble des profils « *A-scan* » simulés sont équivalentes aux statistiques spatiales, il apparaît que la contribution du bruit lors de l'extraction de la surface *3D* des empreintes est négligeable par rapport à la profondeur moyenne d'un sillon, environ égale à 50 μm. L'invention permet ainsi de distinguer correctement par mesures de phase les creux et les sommets des sillons des empreintes digitales. En outre, même dans le cas de performances instrumentales plus faibles, c'est-à-dire pour une résolution axiale faible, il est encore possible d'extraire les sillons de l'empreinte digitale avec une grande précision. L'invention peut permettre de proposer un capteur biométrique OCT performant en imagerie, à plus bas coût que les capteurs connus.

L'invention n'est pas limitée aux exemples qui viennent d'être décrits. L'identification d'empreintes digitales en *3D* requiert des outils plus complexes à mettre en œuvre que les outils traditionnels de correspondance d'images *2D*, comme décrit dans l'article de A. Kumar et C. Kwong, "Toward Contacless, Low-Cost and Acurrate 3D fingerprint Identification", CVPR IEEE Conference, 2013, pp. 3438-3443. Dans l'objectif de pouvoir réutiliser des outils déjà existants, les empreintes digitales *3D* obtenues selon l'invention sont avantageusement transformées en des images *2D* grâce à une méthode de correspondance de texture sur des surfaces *3D*, proche de celle décrite dans l'article de G. Zigelman et al. "Texture mapping using surface flattening via multidimensional scaling", IEEE transactions on Visualization and Computer Graphics, vol. 8, no. 2, 2002. Cette méthode repose sur l'utilisation de l'algorithme de « *Fast Marching* » en anglais, décrit dans l'article de R. Kimmel et J. A. Sethian, "Computing geodesic paths on manifolds", applied mathemathics, Vol. 95, pp. 8431-8435, 1998, et de l'algorithme MDS pour « *Multidimensiononal scaling* » en anglais. En particulier, pour l'aplatissement d'une empreinte digitale 3D, l'algorithme de « *Fast Marching* » est utilisé pour calculer les distances géodésiques à partir d'un maillage triangulaire de son enveloppe moyenne, c'est-à-dire la surface 3D de l'empreinte sans les sillons. L'algorithme de « *Multidimensionnal Scaling* » est appliqué pour transformer la surface *3D* maillée en une image *2D,* sous la contrainte de la minimisation des distorsions des distances géodésiques. Cela permet de préserver au mieux les distances entre les minuties, ce qui est particulièrement intéressant dans le contexte de la biométrie. Différentes images de textures peuvent être projetées sur cette surface 2D aplatie, par exemple l'image de textures en intensité I(x, y), l'image de textures en phase P(x, y), ou l'image de textures en fusion F(x, y). L'invention n'est toutefois pas limitée à un type particulier de méthode pour transformer les images *3D* en images *2D*.

Outre le secteur de la biométrie, l'invention peut être utilisée dans l'étude et l'analyse morphologiques de matériels biologiques, notamment dans le domaine médical, par exemple pour l'imagerie médicale requérant l'étude de la morphologie de surfaces de matériels biologiques situés en profondeur sous la peau.

L'invention peut être utilisée afin de détecter une autre technique de fraude, consistant à effacer l'empreinte digitale externe, ce qui rend inopérante toute technique d'authentification par l'empreinte externe. Si l'on cherche à détecter la fraude, plutôt qu'authentifier la personne, le fait de ne voir aucune empreinte externe mais de voir une empreinte interne permet de déclencher un indicateur de fraude éventuelle.

**Revendications**

1. Procédé d'extraction de caractéristiques morphologiques d'un échantillon de matériel biologique, à savoir des empreintes digitales internes ou externes, utilisant un système d'acquisition par tomographie optique cohérente (OCT) délivrant un signal représentatif de l'échantillon, procédé dans lequel, afin d'extraire des caractéristiques morphologiques de l'échantillon une image contenant des informations d'intensité et une image contenant des informations de phase correspondant au temps de vol de la lumière sont formées à partir au moins du signal délivré par le système d'acquisition et représentatif de l'échantillon, procédé dans lequel, afin de former l'image contenant des informations de phase correspondant au temps de vol de la lumière, on détermine la position d'un pic de réflectivité maximale choisi en fonction des données à extraire, celles de l'empreinte interne ou de l'empreinte externe, d'un profil de réflectivité de la lumière en profondeur (z) établi à partir du signal représentatif délivré par le système d'acquisition, ce profil de réflectivité présentant plusieurs pics de réflectivité maximale, que le premier pic de réflectivité maximale du profil de réflectivité correspondant au pic d'intérêt pour l'empreinte externe, le deuxième pic correspondant au pic d'intérêt pour l'empreinte interne, le procédé étant **caractérisé en ce qu'**une image contenant des informations d'intensité, notamment l'image en intensité (I(x,y)), et l'image contenant des informations de phase, notamment l'image en phase (P(x,y)), sont fusionnées pour former une seule image.

2. Procédé selon la revendication 1, dans lequel on forme une image en phase P(x,y) en utilisant comme référence

12

une enveloppe moyenne Em(x,y) de la surface 3D de l'empreinte considérée, notamment selon P(x,y) = S(x,y) - Em(x,y), où S(x,y) est une image topographique de la surface 3D de l'empreinte considérée, obtenue à partir de la détermination de la position des pics de réflectivité maximale pour chaque A-scan(x,y) du volume tomographique.

3.  Procédé selon l'une quelconque des revendications 1 ou 2, dans lequel la position du pic d'intérêt est estimée puis un filtrage spatial du signal est effectué, notamment un filtrage passe-bande de l'interférogramme dans le domaine spatial, consistant au moins à retenir le signal interférométrique contenu dans une fenêtre centrée sur le pic d'intérêt et de largeur prédéfinie, notamment de l'ordre de grandeur de la résolution axiale du système d'acquisition.

4.  Procédé selon la revendication précédente, dans lequel une transformation est appliquée au signal spatialement filtré afin d'obtenir des informations spectrales, notamment des informations spectrales en intensité et en phase concernant la diffusion enregistrée à l'interface air/doigt dans le cas de l'empreinte digitale externe ou à l'interface épiderme/derme dans le cas de l'empreinte digitale interne

5.  Procédé selon la revendication précédente, dans lequel, afin d'obtenir les informations de phase nécessaires à la formation de l'image en phase, la pente de la phase est calculée par une régression linéaire de la dépendance spectrale de la phase, obtenue à partir des informations spectrales obtenues par transformation du signal spatialement filtré.

6.  Procédé selon l'une quelconque des revendications 1 à 5, dans lequel, pour fusionner l'image contenant des informations d'intensité et l'image contenant des informations de phase, la structure de chaque image est analysée afin d'établir pour chaque image une carte de confiance contenant une valeur de qualité pour chaque pixel, en fonction des pixels voisins.

7.  Procédé selon la revendication précédente, dans lequel chaque pixel de l'image de fusion entre l'image contenant des informations d'intensité et l'image contenant des informations de phase est issu d'une combinaison linéaire des valeurs des pixels correspondants des deux images, pondérées par les valeurs de qualité des cartes de confiance.

8.  Procédé selon la revendication 6, dans lequel l'image de fusion entre l'image contenant des informations d'intensité et l'image contenant des informations de phase est formée en retenant, pour chaque pixel, le pixel de l'image ayant la valeur de qualité la plus élevée.

9.  Procédé selon l'une quelconque des revendications précédentes, dont la revendication 2, dans lequel l'image en phase P(x, y) est projetée sur la surface 3D de l'empreinte interne correspondante.

10. Procédé selon l'une quelconque des revendications 7 à 9, dans lequel, dans le cas où l'échantillon est une empreinte digitale, la valeur de qualité d'un pixel est obtenue à partir de cartes de fiabilité des champs d'orientation des sillons de l'empreinte.

11. Dispositif d'extraction de caractéristiques morphologiques d'un échantillon de matériel biologique, à savoir des empreintes digitales internes ou externes, comprenant un système d'acquisition par tomographie optique cohérente délivrant un signal représentatif de l'échantillon, le dispositif étant configuré pour former, à partir au moins du signal délivré par le système d'acquisition et représentatif de l'échantillon, une image contenant des informations d'intensité, et une image contenant des informations de phase correspondant au temps de vol de la lumière, le dispositif étant configuré pour, afin de former l'image contenant des informations de phase correspondant au temps de vol de la lumière, déterminer la position d'un pic de réflectivité maximale choisi en fonction des données à extraire, celles de l'empreinte interne ou de l'empreinte externe, d'un profil de réflectivité de la lumière en profondeur (z) établi à partir du signal représentatif délivré par le système d'acquisition, ce profil de réflectivité présentant plusieurs pics de réflectivité maximale, le premier pic de réflectivité maximale du profil de réflectivité correspondant au pic d'intérêt pour l'empreinte externe, le deuxième pic correspondant au pic d'intérêt pour l'empreinte interne, le dispositif étant **caractérisé en ce qu'**il est configuré pour fusionner une image contenant des informations d'intensité, notamment l'image en intensité I(x,y), et l'image contenant des informations de phase, notamment l'image en phase P(x,y), afin de former une seule image.

12. Dispositif selon la revendication 11, étant configuré pour former une image en phase P(x,y) en utilisant comme référence une enveloppe moyenne Em(x,y) de la surface 3D de l'empreinte considérée, avec P(x,y) = S(x,y) - Em(x,y), où S(x,y) est l'image topographique de la surface 3D de l'empreinte considérée, obtenue à partir de la détermination de la position du pic de réflectivité maximale d'intérêt (pour chaque A-scan(x,y) du volume tomographique) et pour fusionner l'image en intensité I(x,y) et l'image en phase P(x,y) afin de former une image de fusion F(x,y), une fois la position du pic d'intérêt estimée, effectuer un filtrage spatial du signal, notamment un filtrage passe-bande de l'interférogramme dans le domaine spatial, consistant au moins à retenir le signal interférométrique contenu dans une fenêtre centrée sur le pic d'intérêt et de largeur prédéfinie, notamment de l'ordre de grandeur de la résolution axiale du système d'acquisition, appliquer une transformation au signal spatialement filtré afin d'obtenir des informations spectrales, notamment des informations spectrales en intensité et en phase concernant la diffusion enregistrée à l'interface air/doigt dans le cas d'une empreinte digitale externe ou à l'interface épiderme/derme dans le cas d'une empreinte digitale interne, et pour, afin d'obtenir les informations de phase nécessaires à la formation de l'image en phase, calculer la pente de la phase par une régression linéaire de la dépendance spectrale de la phase, obtenue à partir des informations spectrales obtenues par transformation du signal spatialement filtré.

13. Procédé de détection de fraudes par utilisation de surcouches, dans lequel on met en met œuvre le procédé tel que défini à l'une quelconque 1 à 10 et l'on compare l'empreinte digitale associée au premier pic de réflectivité maximale à celle associée au deuxième pic.

## Patentansprüche

1. Verfahren zur Extraktion morphologischer Merkmale einer Probe von biologischem Material, und zwar internen oder externen Fingerabdrücken, unter Verwendung eines Systems zur Erfassung durch optische Kohärenztomographie (OCT), das ein für die Probe repräsentatives Signal ausgibt, wobei bei dem Verfahren zum Extrahieren der morphologischen Merkmale ein Intensitätsinformationen enthaltendes Bild und ein der Flugzeit des Lichts entsprechende Phaseninformationen enthaltendes Bild ausgehend mindestens von dem vom Erfassungssystem ausgegebenen und für die Probe repräsentativen Signal gebildet werden, wobei bei dem Verfahren zum Bilden des der Flugzeit des Lichts entsprechende Phaseninformationen enthaltenden Bildes die Position eines Peaks maximaler Reflektivität bestimmt wird, der in Abhängigkeit von den zu extrahierenden Daten, denen des internen Abdrucks oder des externen Abdrucks, eines Reflektivitätsprofils des Lichts in der Tiefe (z) gewählt wird, das ausgehend von dem vom Erfassungssystem ausgegebenen repräsentativen Signal erstellt wird, wobei dieses Reflektivitätsprofil mehrere Peaks maximaler Reflektivität aufweist, wobei der erste Peak maximaler Reflektivität des Reflektivitätsprofils dem Peak von Interesse für den externen Abdruck entspricht, wobei der zweite Peak dem Peak von Interesse für den internen Abdruck entspricht, wobei das Verfahren **dadurch gekennzeichnet ist, dass** ein Intensitätsinformationen enthaltendes Bild, insbesondere das Intensitätsbild (I(x,y)), und das Phaseninformationen enthaltende Bild, insbesondere das Phasenbild (P(x,y)) fusioniert werden, um ein einziges Bild zu bilden.

2. Verfahren nach Anspruch 1, bei dem man ein Phasenbild P(x,y) bildet, indem man als Referenz eine mittlere Hüllkurve Em(x,y) der 3D-Oberfläche des betrachteten Abdrucks verwendet, insbesondere gemäß P(x,y) = S(x,y) - Em(x,y), worin S(x,y) ein topographisches Bild der 3D-Oberfläche des betrachteten Abdrucks ist, das ausgehend von der Bestimmung der Position der Peaks maximaler Reflektivität für jeden A-Scan(x,y) des tomografischen Volumens erhalten wird.

3. Verfahren nach einem der Ansprüche 1 oder 2, bei dem die Position des Peaks von Interesse geschätzt wird, dann eine räumliche Filterung des Signals vorgenommen wird, insbesondere eine Bandpassfilterung des Interferogramms im räumlichen Bereich, die mindestens darin besteht, das interferometrische Signal zu berücksichtigen, das in einem Fenster enthalten ist, das auf den Peak von Interesse zentriert ist und von vorgegebener Breite ist, insbesondere in der Größenordnung der axialen Auflösung des Erfassungssystems.

4. Verfahren nach dem vorhergehenden Anspruch, bei dem eine Transformation auf das räumlich gefilterte Signal angewandt wird, um spektrale Informationen zu erhalten, insbesondere spektrale Intensitäts- und Phaseninformationen bezüglich der Streuung, die im Fall des externen Fingerabdrucks an der Luft/Finger-Grenzfläche oder im Fall des internen Fingerabdrucks an der Epidermis/Dermis-Grenzfläche verzeichnet wird.

5. Verfahren nach dem vorhergehenden Anspruch, bei dem zum Erhalten der Phaseninformationen, die zur Bildung des Phasenbildes erforderlich sind, die Phasensteilheit durch eine lineare Regression der spektralen Abhängigkeit der Phase berechnet wird, die anhand der durch Transformation des räumlich gefilterten Signals erhaltenen spektralen Informationen erhalten wird.

6. Verfahren nach einem der Ansprüche 1 bis 5, bei dem zum Fusionieren des Intensitätsinformationen enthaltenden Bildes und des Phaseninformationen enthaltenden Bildes die Struktur jedes Bildes analysiert wird, um für jedes Bild eine Konfidenzkarte zu erstellen, die einen Qualitätswert für jedes Pixel in Abhängigkeit von den Nachbarpixeln enthält.

7. Verfahren nach dem vorhergehenden Anspruch, bei dem jedes Pixel des Fusionsbildes zwischen dem Intensitätsinformationen enthaltenden Bild und dem Phaseninformationen enthaltenden Bild aus einer linearen Kombination der Werte der entsprechenden Pixel der beiden Bilder, gewichtet durch die Qualitätswerte der Konfidenzkarten, hervorgegangen ist.

8. Verfahren nach Anspruch 6, bei dem das Fusionsbild zwischen dem Intensitätsinformationen enthaltenden Bild und dem Phaseninformationen enthaltenden Bild gebildet wird, indem für jedes Pixel das Pixel des Bildes berücksichtigt wird, das den höchsten Qualitätswert hat.

9. Verfahren nach einem der vorhergehenden Ansprüche, darunter Anspruch 2, bei dem das Phasenbild P(x, y) auf die 3D-Oberfläche des entsprechenden internen Abdrucks projiziert wird.

10. Verfahren nach einem der Ansprüche 7 bis 9, bei dem in dem Fall, in dem die Probe ein Fingerabdruck ist, der Qualitätswert eines Pixels ausgehend von Zuverlässigkeitskarten der Ausrichtungsfelder der Rillen des Abdrucks erhalten wird.

11. Vorrichtung zur Extraktion morphologischer Merkmale einer Probe von biologischem Material, und zwar internen oder externen Fingerabdrücken, umfassend ein System zur Erfassung durch optische Kohärenztomographie, das ein für die Probe repräsentatives Signal ausgibt, wobei die Vorrichtung dazu ausgelegt ist, ausgehend mindestens von dem vom Erfassungssystem ausgegebenen und für die Probe repräsentativen Signal ein Intensitätsinformationen enthaltendes Bild und ein der Flugzeit des Lichts entsprechende Phaseninformationen enthaltendes Bild zu bilden, wobei die Vorrichtung dazu ausgelegt ist, zum Bilden des der Flugzeit des Lichts entsprechende Phaseninformationen enthaltenden Bildes die Position eines Peaks maximaler Reflektivität zu bestimmen, der in Abhängigkeit von den zu extrahierenden Daten, denen des internen Abdrucks oder des externen Abdrucks, eines Reflektivitätsprofils des Lichts in der Tiefe (z) gewählt wird, das ausgehend von dem vom Erfassungssystem ausgegebenen repräsentativen Signal erstellt wird, wobei dieses Reflektivitätsprofil mehrere Peaks maximaler Reflektivität aufweist, wobei der erste Peak maximaler Reflektivität des Reflektivitätsprofils dem Peak von Interesse für den externen Abdruck entspricht, wobei der zweite Peak dem Peak von Interesse für den internen Abdruck entspricht, wobei die Vorrichtung **dadurch gekennzeichnet ist, dass** sie dazu ausgelegt ist, ein Intensitätsinformationen enthaltendes Bild, insbesondere das Intensitätsbild I(x,y), und das Phaseninformationen enthaltende Bild, insbesondere das Phasenbild P(x,y), zu fusionieren, um ein einziges Bild zu bilden.

12. Vorrichtung nach Anspruch 11, die dazu ausgelegt ist, ein Phasenbild P(x,y) zu bilden, indem als Referenz eine mittlere Hüllkurve Em(x,y) der 3D-Oberfläche des betrachteten Abdrucks verwendet wird, mit P(x,y) = S(x,y) - Em(x,y), worin S(x,y) das topographische Bild der 3D-Oberfläche des betrachteten Abdrucks ist, das ausgehend von der Bestimmung der Position des Peaks maximaler Reflektivität von Interesse erhalten wird (für jeden A-Scan(x,y) des tomographischen Volumens), und das Intensitätsbild I(x,y) und das Phasenbild P(x,y) zu fusionieren, um ein Fusionsbild F(x,y) zu bilden, nachdem die Position des Peaks von Interesse geschätzt wurde, eine räumliche Filterung des Signals vorzunehmen, insbesondere eine Bandpassfilterung des Interferogramms im räumlichen Bereich, die darin besteht, das interferometrische Signal zu berücksichtigen, das in einem Fenster enthalten ist, das auf den Peak von Interesse zentriert ist und von vorgegebener Größe ist, insbesondere in der Größenordnung der axialen Auflösung des Erfassungssystems, eine Transformation auf das räumlich gefilterte Signal anzuwenden, um spektrale Informationen zu erhalten, insbesondere spektrale Intensitäts- und Phaseninformationen bezüglich der Streuung, die im Fall eines externen Fingerabdrucks an der Luft/Finger-Grenzfläche oder im Fall eines internen Fingerabdrucks an der Epidermis/Dermis-Grenzfläche verzeichnet wird, und zum Erhalten der Phaseninformationen, die zum Bilden des Phasenbildes erforderlich sind, die Phasensteilheit durch eine lineare Regression der spektralen Abhängigkeit der Phase zu berechnen, die ausgehend von spektralen Informationen erhalten wird, die durch Transformation des räumlich gefilterten Signals erhalten werden.

13. Verfahren zur Erkennung von Betrügereien durch Verwendung von Deckschichten, bei dem das Verfahren nach einem 1 bis 10 eingesetzt wird und der dem ersten Peak maximaler Reflektivität zugeordnete Fingerabdruck mit dem, der dem zweiten Peak zugeordnet ist, verglichen wird.

**Claims**

1. A method for extracting morphological characteristics from a sample of biological material, comprising an internal and an external print, using an optical coherence tomography (OCT) acquiring system delivering a signal representative of the sample, the method comprising forming based at least on the signal representative of the sample an image containing intensity data and an image containing phase data corresponding to the time of flight of light, in order to extract morphological characteristics of the sample, wherein, in order to form the image containing phase data corresponding to the time of flight of the light, a position of a maximum reflectivity peak is determined based on the data to be extracted, the ones of the internal print or of the external print, of a profile of reflectivity of the light with depth established from the signal delivered by the acquiring system, said profile of reflectivity comprising several maximum reflectivity peaks, the first maximum reflectivity peak of the profile of reflectivity corresponding to the peak of interest for the external print, the second peak corresponding to the peak of interest for the internal print, method **characterized in that** an image containing intensity data, especially the intensity image I(x,y), and the image containing phase data, especially the phase image P(x,y), are fused to form a single image.

2. The method according to claim 1, wherein a phase image is formed using as reference an average envelope Em(x,y) of a 3D surface of the corresponding print, especially with P(x,y) = S(x,y) - Em(x,y), where S(x,y) is a topographical image of the 3D surface of the corresponding print, obtained from the determination of the position of the maximum reflectivity peaks for A-scans (x,y) of a tomographic volume.

3. The method according to any one of claim 1 or 2, wherein the position of the peak of interest is estimated and then spatial filtering is carried out on the signal, especially a passband filtering of the reflectogram in the spectral domain, the filtering comprising retaining an interferometric signal contained in a window centered on the peak of interest and of a predefined width, especially of an order of magnitude of an axial resolution of the acquiring system.

4. The method of the preceding claim, wherein a transformation is applied to the spatially filtered signal in order to obtain spectral data, especially spectral data in intensity and phase relating to a scattering recorded at an air/finger interface in the case of the external print or at an epidermal/dermal interface in the case of the internal print.

5. The method according to the preceding claim, wherein, in order to obtain phase data to form the phase image, a slope of the phase is calculated by linear regression of the spectral dependence of the phase, which is obtained from spectral data obtained by transforming the spatially filtered signal.

6. The method according to any one of claims 1 to 5, wherein, to fuse the image containing intensity data and the image containing phase data, a structure of each image is analysed in order to establish, for each image, a confidence map containing, for each pixel, a quality value, depending on neighbouring pixels.

7. The method according to the preceding claim, wherein each pixel of the image fused from the image containing intensity data and the image containing phase data is the result of a linear combination of the values of corresponding pixels of the two images, weighted by the quality values of the confidence maps.

8. The method according to claim 6, wherein the image fused from the image containing intensity data and the image containing phase data is formed by retaining, for each pixel, the pixel of the image having a highest quality value.

9. The method according to any one of the preceding claims, including claim 2, wherein the phase image P(x,y) is projected onto the 3D surface of the corresponding internal print.

10. The method according to any one of claims 7 to 9, the sample being a fingerprint, the quality value of a pixel is obtained from print valley orientation field reliability maps.

11. A device for extracting morphological characteristics from a sample of biological material comprising internal and external prints, comprising an optical coherence tomography acquiring system delivering a signal representative of the sample, the device being configured to form, from at least the signal delivered by the acquiring system and representative of the sample, an image containing intensity data and an image containing phase data corresponding to the time of flight of light, the device being configured, in order to form the image containing phase data corresponding to the time of flight of the light, to determine a position of a reflectivity peak of interest of a profile of reflectivity of the light with depth (z), said profile being established from the signal delivered by the acquiring system, the reflectivity profile comprising several maximum reflectivity peaks, the first maximum reflectivity peak corresponding to the peak of interest for the external print, the second peak corresponding to the peak of interest for the internal print, device being characterized for fusing an image containing intensity data, especially the intensity image I(x,y), and the image containing phase data, especially the phase image P(x,y), in order to form a single image.

12. Device according to Claim 11, being configured to form a phase image P(x,y) using as reference an average envelope Em(x,y) of the 3D surface of the print, where P(x,y) = S(x,y) - Em(x,y), where S(x, y) is a topographic image of the 3D surface of the print, which image is obtained from the determination of the position of the maximum reflectivity peak of interest for each A-scan(x,y) of a tomographic volume, and to fuse the intensity image I(x,y) and the phase image P(x,y) in order to form a fused image F(x,y), and once the position of the peak of interest has been estimated, to carry out spatial filtering on the signal, the filtering consisting at least in retaining the interferometric signal contained in a window centred on the peak of interest and of a predefined width and to apply a transformation to the spatially filtered signal in order to obtain spectral data relating to the scattering recorded at an air/finger interface in the case of an external print or at the epidermal/dermal interface in the case of an internal print, and, in order to obtain the phase data required to form the phase image, to calculate the slope of the phase by linear regression of a spectral dependence of the phase, which is obtained from spectral data obtained by transforming the spatially filtered signal.

13. Method for detecting fraud using overlayers, in which the method as defined in any one of claims 1 to 10 is implemented and the fingerprint associated with the first reflectivity peak is compared with that associated with the second peak.

épiderme

derme

Empreinte interne

Empreinte externe

## Fig. 1

(a)

(b)

ETAT de L'ART

## Fig. 2

Fig. 3

Fig. 4

Gouttes
d'eau

Empreinte
digitale

Fig. 5

ETAT de L'ART
Fig. 6

Epiderme

Derme papillaire

Zone de jonction

Derme

ETAT de L'ART
Fig. 7

ETAT de L'ART
Fig. 8

niveau de la
sonde du dispositif

Empreinte
digitale

Enveloppe
moyenne

## Fig. 9

10

12

Miroir

Miroir
partiel

Réflexlions
èchantillon

11

Source á
Balayage

13

$\Delta L_1$ $\Delta L_2$

$\lambda$

14

PD

Interféromètre
de Michelson

Franges
d'interférence

temps

+

FFT

Signal OCT

## Fig. 10

(a)

(b)

# Fig. 11

(a)

(b)

# Fig. 12

(a)    (b)

## Fig. 13

(a)    (b)

## Fig. 14

Fig. 15

FRR

FAR

- - - Empreinte interne - Phase
- - Empreinte interne - Intensité moyenne
— Empreinte interne - Fusion
- - - - Empreinte externe - Phase

Fig. 16

Fig. 17

Fig. 18

(a)                                    (b)

# Fig. 19

# Fig. 20

Fig. 21

(a)        (b)        (c)

Fig. 22

101

103
104
105

Mesure OCT

TF

Interférogramme U (ν)
(Domaine Spectral)

Réflectogramme u (τ)
(Domaine temporel)

Estimation de la position
$\tau_0$ du pic d'intérêt par
mesure directe de
temps de vol

102

107

TF inverse

Filtrage passe-bande
autour du pic d'intérêt

108

Interférogramme filtré $U_f(\nu)$
(Domaine spectral)

109

Hilbert

106

Arg

Signal complexe $\widehat{U_f}(\nu)$
(Domaine spectral)

Phase spectrale $\varphi_m(\nu)$
(Domaine spectral)

Coeff. directeur de la
régression linéaire
de $\frac{1}{2\pi}\varphi_m(\nu)$

110

Position $\tau_0'$ du pic d'intérêt

112

111

Fig. 23

101
104

Pour chaque position
(X,Y) de la sonde,
mesure OCT

Réflectogramme (Ascan (x,y))

115

Mesure de la position du pic
d'intérêt via la phase spectrale

1$^{er}$ pic

Surface 3D $S_{externe}$ (x, y)
de l'empreinte externe

110

2$^{ème}$ pic

Surface 3D $S_{externe}$ (x, y)
de l'empreinte interne

116

Fig. 24

Surface 3D et image topographique S(x,y) associée

Soustraction

153

150

Filtrage passe-bas 2D de la carte topographique associée

152

Enveloppe moyenne E$_m$(x,y)

151

Moyennage des niveaux d'intensité de la zone de jonction, autour de l'enveloppe moyenne

160

155

Image de textures en Phase P(x,y)

Image de textures en Intensité I(x,y)

161

Post-traitements (égalisation d'histogrammes et amélioration de contraste)

154

Fusion

166

165

Image de texture de Fusion F(x,y)

Projection des images de textures sur les surfaces 3D d'empreintes correspondantes

167

Aplatissement des surfaces 3D d'empreintes texturées

168

Images aplaties d'empreintes digitales externe et interne.

169

Fig. 25

Surface 3D d'empreinte

Enveloppe moyenne

152

$S(x, y)$

Filtrage passe-bas 2D

$E_m(x, y)$

153 — Soustraction

154

Post-traitements (égalisation d'histogrammes et amélioration de contraste)

155

Image de textures en phase $P(x,y)$

Fig. 26

## RÉFÉRENCES CITÉES DANS LA DESCRIPTION

**Documents brevets cités dans la description**

- US 20140241596 A

- US 2015016127 A

**Littérature non-brevet citée dans la description**

- **A. F. FERCHER et al.** Optical Coherence Tomography - Principles and Applications. *Reports on progress in physics,* 2003, 239-303
- **A. BOSSEN et al.** Internai Fingerprint identification with optical coherence tomography. *IEEE Photonics technology letters,* 2010, vol. 22 (7
- **M. LIU et al.** Biometric mapping of fingertip eccrine glands with optical coherence tomography. *IEEE photonics technology letters,* 2010, vol. 22 (22
- **R. CAPPELLI et al.** Performances evaluation of fingerprint vérification systems. *IEEE transactions on pattern analysis and maching intelligence,* 2006, vol. 28 (1
- **L.C. JAIN et al.** Intelligent biometric techniques in fingerprint and face recognition. CRC press, 1999, vol. 10
- **KHUTLANG.** Novelty Detection-Based Internai Fingerprint Segmentation. *Optical Coherence Tomography Images 2014 Second International Symposium on Computing and Networking Rethabile*
- *Biometric Mapping of Fingertip Eccrine Glands With Optical Coherence Tomography IEEE Photonics Technology Letters,* 15 Novembre 2010, vol. 22 (22
- *Impact of Quality-Based Fusion Techniques for Video-Based Iris Recognition at a Distance Nadia Othman and Bernadette Dorrizi IEEE TRANSACTIONS on INFORMATION FORENSICS AND SECURITY,* Août 2015, vol. 10 (8

- **J. ZHOU ; J. GU.** A Model-based for the computation of fingerprint's orientation field. *IEEE Transactions on Image Processing,* 2004, vol. 13 (6
- **M. S. KHALIL.** Deducting fingerprint singular points using orientation field reliability. *First conference on robot, vision and signal processing,* 2011, 234-286
- **L. HONG et al.** Fingerprint image enhancement: algorithm and performance evaluation. *IEEE Transactions on Pattern Analysis and Machine Intelligence,* 1998, vol. 20 (8
- **C. SOUSEDIK et al.** Volumetric Fingerprint Data Analysis using Optical Coherence Tomography. *BIOSIG Conference,* 2013, 1-6
- **C. SOUSEDIK ; C. BUSH.** Quality of fingerprint scans captured using Optical Coherence Tomography. *IJCB Conference,* 2014, 1-8
- **A. KUMAR ; C. KWONG.** Toward Contacless, Low-Cost and Acurrate 3D fingerprint Identification. *CVPR IEEE Conference,* 2013, 3438-3443
- **G. ZIGELMAN et al.** Texture mapping using surface flattening via multidimensional scaling. *IEEE transactions on Visualization and Computer Graphics,* 2002, vol. 8 (2
- **R. KIMMEL ; J. A. SETHIAN.** Computing geodesic paths on manifolds. *applied mathemathics,* 1998, vol. 95, 8431-8435